# EUROPEAN PATENT APPLICATION

(11) **EP 4 317 141 A1**
(43) Date of publication of application: **07.02.2024**
(21) Application number: 22382756.9
(22) Date of filing: 04.08.2022
(51) Int. Cl.: C07D 265/30, C07C 215/08, C07C 215/10

(54) **PROCESS AND INTERMEDIATES FOR THE PREPARATION OF VILOXAZINE AND OTHER 2-SUBSTITUTED MORPHOLINE DERIVATIVES**

(71) Applicant: Curia Spain S.A.U., 47151 Boecillo, Valladolid (ES)
(72) Inventor: ENCINAS MADRAZO, Soledad, 47151 Boecillo, Valladolid (ES); RODRÍGUEZ FERRER, Patricia, 47151 Boecillo, Valladolid (ES)
(74) Representative: ABG Intellectual Property Law, S.L.

(57) **Abstract**

The present invention relates to an efficient and industrially applicable process for the preparation of 2-substituted morpholine derivatives such as viloxazine and to intermediates useful therefor.

## Description

### Field of the Invention

The invention belongs to the field of drug synthesis, and more particularly relates to an efficient and industrially applicable process for the preparation of 2-substituted morpholine derivatives such as viloxazine and to intermediates useful therefor.

### Background of the Invention

Viloxazine is the international non-proprietary name (INN) or common name of 2-[(2-ethoxyphenoxy)methyl]morpholine, a well-known racemic morpholine derivative with CAS No. 46817-91-8, originally discovered by Imperial Chemical Industries in the late 1960s, when its scientists tried to synthesize compounds with propranolol-like structures with CNS modulating properties. It acts as a selective norepinephrine reuptake inhibitor (SNRI) and has the following chemical structure:

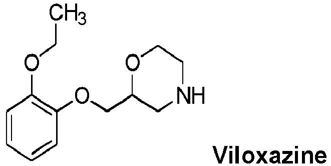

Viloxazine hydrochloride was approved in April 2021 by the US Food and Drug Administration (FDA) under the trade name Qelbree^{®} for the treatment of attention-deficit/hyperactivity disorder (ADHD) in children and adolescents. However, it was previously marketed from the 1970s until 2002 (when it was withdrawn for commercial reasons unrelated to safety and efficacy) as an antidepressant in Europe under different trade names (Viloxazin^{®}, Emovit^{®}, Viloxazina^{®}, Viloxazinum^{®}, Vivalan^{®}, Vivarin^{®}, Vivarint^{®}, Vicilan^{®}) before being repurposed as a treatment for ADHD in the USA.

In spite of being a commercial product, in general the processes described in the state of the art for its synthesis can be considered as not satisfactory, mainly because most of them have low yields and/or some of the intermediates or reagents used are genotoxic in nature. Thus, it is of utmost importance to guarantee that this active pharmaceutical ingredient can be obtained in high purity.

Viloxazine was described for the first time in BE 708557A (also published as US 3714161), wherein it is prepared by reacting o-ethoxyphenol with epichlorhydrin in basic conditions, and the resulting epoxide intermediate 1-(2-ethoxyphenoxy)-2,3-epoxypropane is heated with benzylamine followed with treatment with chloroacetyl chloride. The resulting cyclic amide is reduced with lithium aluminum hydride, followed by hydrogenation to remove the benzyl group to yield viloxazine, which optionally may be isolated as the hydrochloride (see also Mallion KB, Todd AH, Turner RW, Bainbridge JG, Greenwood DT, Madinaveitia J, et al. 2-(2-Ethoxyphenoxymethyl)tetrahydro-1,4-oxazine hydrochloride, a potential psychotropic agent. Nature 1972; 238: 157-8).

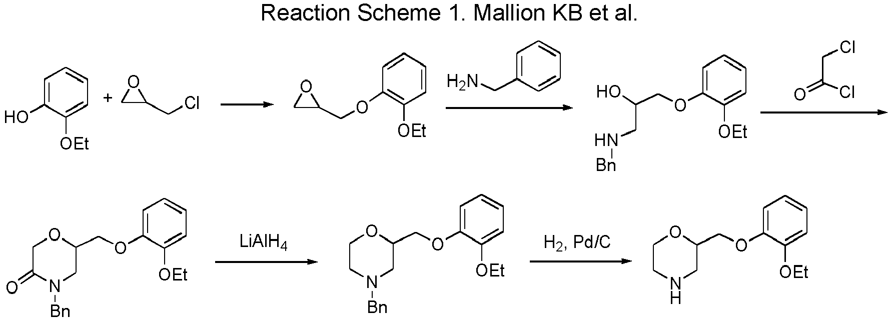

However, this pioneer method of synthesizing viloxazine is unsatisfactory since it involves a long synthesis with poor yields (20% overall based on propanolamine intermediate) as it was reported afterwards by the same authors (Greenwood DT, Mallion KB, Todd AH, Turner RW. 2-Aryloxymethyl-2,3,5,6-tetrahydro-1,4-oxazines, a new class of antidepressants. J Med Chem. 1975; 18(6): 573-7).

US 3857839 and US 3712890 relate to a process to prepare viloxazine, wherein the epoxide intermediate, 1-(2-ethoxyphenoxy)-2,3-epoxypropane, is reacted with a 2-aminoethyl derivative (e.g. 2-aminoethyl hydrogen sulfate) in ethanol in the presence of sodium hydroxide to form viloxazine free base, that may be isolated as viloxazine HCI. Example 10 of US 3857839 describes a variant of the process in which the intermediate addition product is isolated before being cyclized. 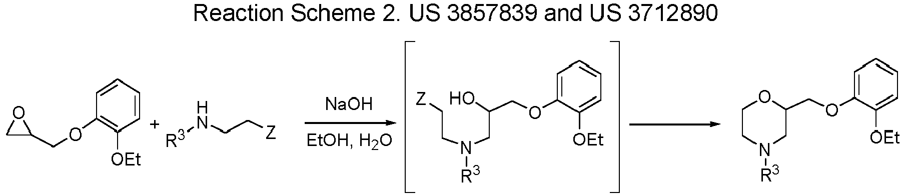 wherein R³ may be H or benzyl, among others, and Z represents a displaceable radical such as an halogen atom or a sulfonyloxy radical.

In this case, low yields are reported, together with the use of genotoxic reactants such as for example: epichlorohydrin, alkylating agents or large amounts of NH₂-CH₂-CH₂-SO₃H to form the aminoalcohol intermediate.

WO 2011130194 relates to methods for producing viloxazine salts and polymorphs thereof. This patent application acknowledges that the foregoing methods of synthesizing viloxazine suffer from a number of deficiencies, such as low reaction yield and unacceptably large amount of impurities in the resulting product. Effective elimination or removal of impurities, especially those impurities possessing genotoxicity or other toxicities, is critical to render safe pharmaceutical products. For example, certain reagents traditionally utilized in viloxazine preparation, such as epichlorohydrin and 2-aminoethyl hydrogen sulfate, present a special problem due to their toxicity. Nevertheless, WO 2011130194 in reality makes also use of the two processes that had been already disclosed in previous documents (see example 1 and examples 17-18, respectively) and in essence, this patent application is aimed at improving the preparation of the initial epoxide and the work-up and isolation of the salts of viloxazine.

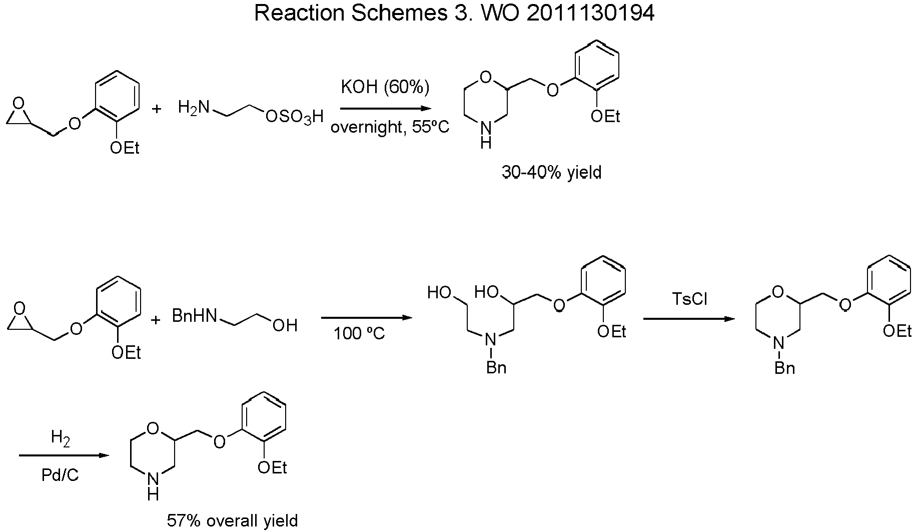

Another approach for the preparation of viloxazine involves an intramolecular C-O bond formation of alkanol-epoxide mediated by boron trifluoride as key transformation in a relatively long (7 steps) total synthesis (Gosh P, Deka M, Saikia, A. Lewis Acid Mediated Intramolecular C-O Bond Formation of Alkanol-Epoxide Leading to Substituted Morpholine Derivatives: Total Synthesis of (+) - Viloxazine. Tetrahedron 2016, 72, 690-698).

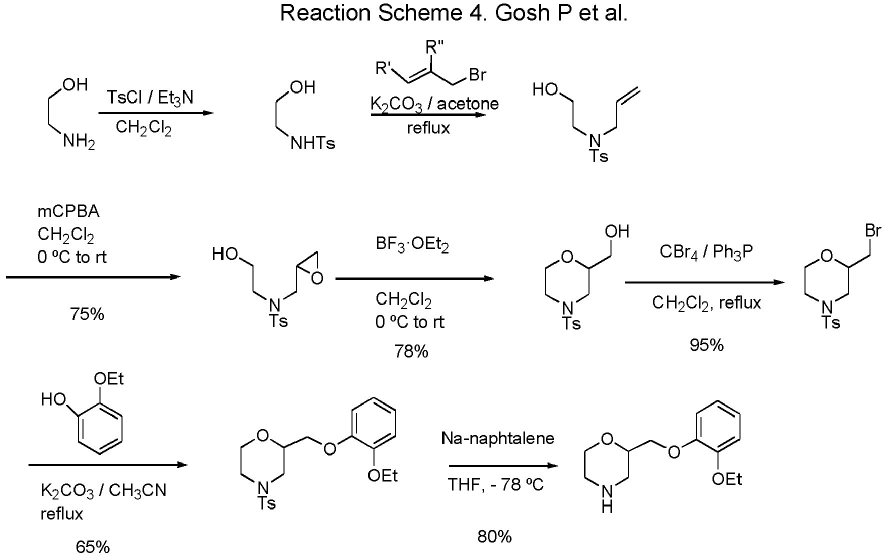

The Imperial Chemical Industries, in connection with the synthesis of antidepressant compounds, carried out further studies towards the preparation of suitable morpholine intermediates functionalised in the 2-position (Loftus F, The Synthesis of Some 2-Substituted Morpholines, Synthetic Communications, 10(1), 59-73 (1980)). N-Benzyl-2-chloromethylmorpholine (1) was prepared by dissolving N-benzylethanolamine in an excess of epichlorohydrin and the solution was stirred at 40 °C. Then, the excess epichlorohydrin was removed by distillation under reduced pressure to leave the intermediate amine (numbered as 8 in the paper and as 2 herein) as a gum, which was dissolved in 98% sulfuric acid and the solution was heated rapidly to 150 °C. Work-up of the reaction mixture afforded N-benzyl-2-chloromethylmorpholine (3) as a colourless oil in a 79% yield.

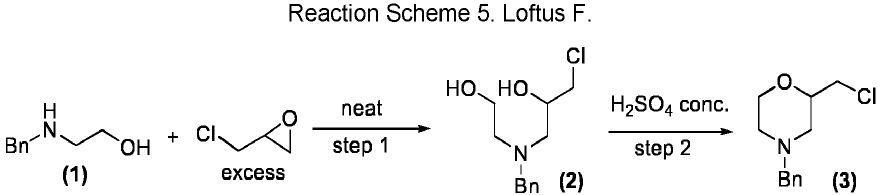

This last route developed by the Imperial Chemical Industries to prepare N-benzyl-2-chloromethylmorpholine or related benzyl substituted derivatives has been repeated in more recent research, although not with the aim of synthesizing viloxazine. See for instance WO 2015092804 (preparation 12, step (ii)) or Audouze K, Nielsen EØ, Peters D. New series of morpholine and 1,4-oxazepane derivatives as dopamine D4 receptor ligands: synthesis and 3D-QSAR model. J Med Chem. 2004; 47(12): 3089-3104 (preparation of compound (6c), Procedure E).

However, the procedures described in the state of the art to obtain the morpholine intermediate (3) by the sequence shown in Reaction Scheme 5 above suffer from two important shortcomings:
- The initial ethanolamine derivative (1) is blended in neat conditions (without any solvent) with an excess of epichlorohydrin (1.1 to 10 eq), which is genotoxic in nature, and such an excess of epichlorohydrin must be removed by distillation once step 1 is complete or, alternatively, column chromatography is necessary at the end of the process. Purification by distillation might not reproducibly yield the product in a sufficiently pure state whereas purification by column chromatography is also unsuitable, or at least undesirable, for industrial scale manufacture since an extremely large amount of solvent is required which makes the process expensive and is disadvantageous because of environmental protection.
- Concentrated sulfuric acid in excess is added to the mixture after step 1 resulting in a highly exothermic process. An exothermic reaction can lead to thermal runaway, which begins when the heat produced by the reaction exceeds the heat removed and the reaction goes out of control. Further, the scale on which the reaction is carried out may have a significant effect on the likelihood of runaway since the heat produced increases with the volume of the reaction mixture, whereas the heat removed depends on the surface area available for heat transfer. Thus, whenever possible, very exothermic processes must be avoided at an industrial scale.

In summary, the methods disclosed in the prior art for the preparation of viloxazine or intermediates suitable therefor are not industrially applicable for different reasons (low yield, too long, presence of impurities with undesirable pharmacological properties, risk of runaway reactions in scaling up, etc). It is therefore necessary to develop a new process for obtaining key intermediates in the synthesis of viloxazine and related compounds which overcome all or part of the problems associated with the known processes belonging to the state of the art.

### Summary of the Invention

The present invention solves the aforementioned need by the provision of a simple and industrially applicable process for the preparation of 2-substituted morpholine derivatives such as viloxazine and to intermediates useful therefor. Specifically, the process provided in the present invention allows obtaining a 2-chloromethylmorpholine intermediate of formula general (III) in high yield and purity under mild and more appropriate conditions in comparison to the common procedure in the state of the art to prepare these compounds. The subsequent conversion of the 2-chloromethylmorpholine intermediate into viloxazine or related compounds is straightforward, thus making this methodology practical for its implementation in the industry. The present invention also provides novel salts of N-benzyl-2-chloromethylmorpholine which are encompassed within general formula (III).

After extensive research, the present inventors realized that the process disclosed by Loftus, and followed by other authors, to prepare N-benzyl-2-chloromethylmorpholine (see Reaction Scheme 5) cannot be scaled up in an industrial manner *inter alia* due to:
- Epichlorohydrin (genotoxic) coming from step 1 always remain in some quantity in the reaction mixture before the addition of the concentrated sulfuric acid;
- Epichlorohydrin in contact with strong acids (e.g. sulfuric acid) can cause explosive polymerization (Wiley Guide to Chemical Incompatibilities, pag. 498);
- The use of sub-stoichiometric quantities of epichlorohydrin to avoid its presence once the reaction is over favors the formation of the dimer impurity **(8):** 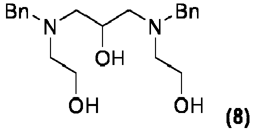
- The intermediate 1-chloro-2-propanol-N derivative (compound **(2)** in Reaction Scheme 5) is unstable, giving raise to by-products, mainly to the azetidine **(6)** and the epoxide **(7),** and therefore it cannot be stored in solution for a long time or isolated by solvent distillation without generating unwanted impurities: 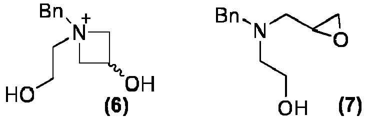
- The addition of concentrated sulfuric acid, even slowly and at low temperature, is very exothermic and difficult of being controlled, especially at large scale. A potential runaway reaction may thus occur in the presence of concentrated H₂SO₄ which is very corrosive.
- The use of concentrated sulfuric acid implies low reaction volumes, difficult to stir and an exothermic work-up with the formation of tars.

As a conclusion, a process as described by Loftus, among others, is not adequate for industrial purposes.

Thus, the present inventors conducted extensive experimentation with an intention of improving the preparation of N-benzyl-2-chloromethylmorpholine and other N-substituted-2-chloromethylmorpholines with the aim of implementing in the industrial preparation of 2-substituted morpholine derivatives and more specifically viloxazine. As a result of this research, the inventors have surprisingly found a new methodology based on the following key findings:
- The use of an organic solvent together with a Lewis acid in the first step (preparation of intermediate (**2**)) allows a better handling of the reaction and a more stable media, without significant secondary reactions or formation of impurities, as compared with the solvent free (neat) reaction proposed in the state of the art;
- Contrary to what was held in the state of the art (see for example US 2777846), the sulfuric acid cyclization reaction to obtain morpholine rings is not a dehydration reaction that requires an anhydrous media by using concentrated sulfuric acid or oleum (fuming sulfuric acid), but it also works in the presence of water;
- Thus, a H₂SO₄/water mixture may be used to extract the intermediate (**2**) from the reaction mixture and to carry out the cyclization in step 2;
- This mixture of H₂SO₄/water allows, on one hand, the elimination of the non-basic impurities including the remaining genotoxic epichlorohydrin (an excess of epichlorohydrin may thus be used in step 1, if desired);
- Further, the exothermic behavior is managed appropriately when the intermediate (**2**) is extracted with a H₂SO₄/water mixture and concentrated sulfuric acid is added to perform the second step to afford the morpholine compound (3) with very good yields and a very clean profile.

Altogether, with this experimental procedure, it is possible to control the exothermic behavior that occurs when large quantities of reagents and reactants are handled and still achieve the desired product with lesser impurities. In addition, the reaction mixture is also less dense and better workable. As the skilled person will understand, the process is not limited to N-benzylethanolamine (**1**) but other amino protected ethanolamine compounds may also be used.

Thus, in a first aspect, the invention is directed to a process for preparing a compound of general formula (III) or a salt or solvate thereof 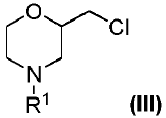 wherein R¹ is an amino protecting group,
said process comprising:
a) reacting epichlorohydrin with a compound of general formula (I) or a salt or solvate thereof 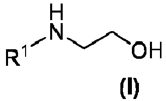 wherein R¹ is an amino protecting group,
   in the presence of a Lewis acid and an organic solvent to obtain a reaction mixture comprising a compound of general formula (II) or a salt or solvate thereof 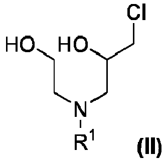 wherein R¹ is an amino protecting group,
b) mixing the reaction mixture with an aqueous solution of sulfuric acid to obtain a water-sulfuric phase comprising a sulfate salt of the compound of general formula (II); and
c) mixing the water-sulfuric phase comprising a sulfate salt of the compound of general formula (II) with concentrated sulfuric acid to obtain the compound of general formula (III) or a salt or solvate thereof.

Compounds of formula (III), and salts or solvates thereof, are intermediates in the synthesis of viloxazine and other 2-substituted morpholine compounds. Thus, the final making of viloxazine and related derivatives through the process provided herein for preparing a compound of general formula (III) or a salt or solvate thereof is also encompassed within the present invention.

In a further aspect, the invention is directed to a salt of 4-benzyl-2-(chloromethyl)morpholine, said salt being selected from the oxalate, fumarate or tosylate salt.

These aspects and preferred embodiments thereof are additionally also defined hereinafter in the detailed description and in the claims.

### Brief description of Drawings

To better understand the invention, its objects and advantages, the following figures are attached to the specification in which the following is depicted:

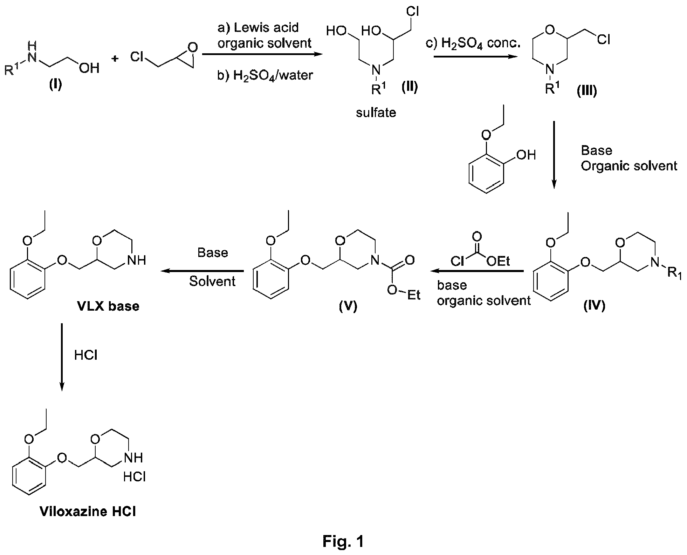

**Figure 1** shows a general scheme of a process for preparing viloxazine HCI according to the present invention.

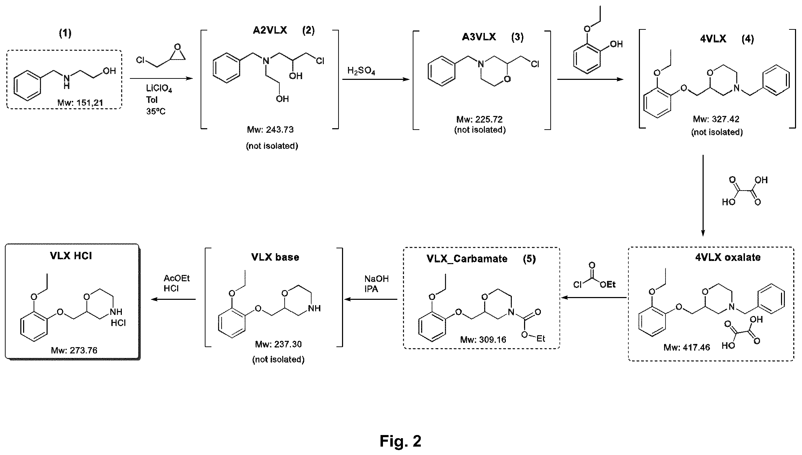

**Figure 2** shows a scheme of a process for preparing viloxazine HCI according to an embodiment of the present invention starting from N-benzylethanolamine (**1**) (A3VLX free base, i.e. the free base of compound (**3**), is used for further transformation into 4VLX, i.e. compound (**4**)).

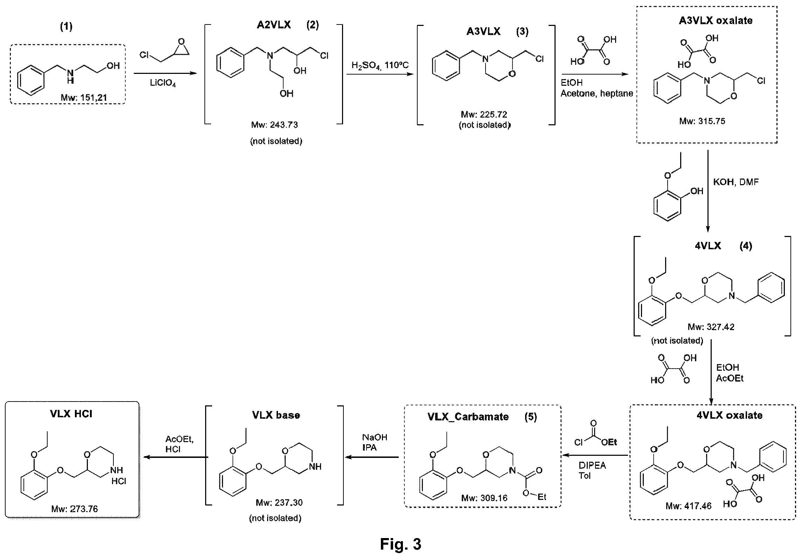

**Figure 3** shows a scheme of a process for preparing viloxazine HCI according to an embodiment of the present invention starting from N-benzylethanolamine (**1**) (A3VLX oxalate salt, i.e. the oxalate salt of compound (**3**), is used for further transformation into 4VLX, i.e. compound (**4**)).
**Figure 4** shows ¹H NMR of 4-benzyl-2-(chloromethyl)morpholine oxalate (oxalate salt of compound (**3**) or A3VLX oxalate), the compound of example 5.
**Figure 5** shows differential scanning calorimetry (DSC) of 4-benzyl-2-(chloromethyl)morpholine oxalate (oxalate salt of compound (**3**) or A3VLX oxalate, the compound of example 5.
**Figure 6** shows ¹H NMR of 4-benzyl-2-(chloromethyl)morpholine fumarate (fumarate salt of compound (**3**) or A3VLX fumarate), the compound of example 6.
**Figure 7** shows DSC of 4-benzyl-2-(chloromethyl)morpholine fumarate (fumarate salt of compound (**3**) or A3VLX fumarate), the compound of example 6.
**Figure 8** shows ¹H NMR of 4-benzyl-2-(chloromethyl)morpholine tosylate (tosylate salt of compound (**3**) or A3VLX tosylate), the compound of example 7.
**Figure 9** shows DSC of 4-benzyl-2-(chloromethyl)morpholine tosylate (tosylate salt of compound (**3**) or A3VLX tosylate), the compound of example 7.
**Figure 10** shows X-ray powder diffraction (XRPD) pattern of viloxazine obtained according to an embodiment of the present invention.

### Detailed Description of the Invention

The present inventors have developed a new process for the preparation of 2-substituted morpholine derivatives such as viloxazine and to intermediates useful therefor. The process of the present invention is simple, reproducible and is well suited for industrial scale.

### Definitions

The skilled person knows that numerical values relating to measurements are subject to measurement errors which place limits on their accuracy. Where terms such as "about" or "approximately" are applied to a particular value (e.g. "about 200 °C" or "approximately 200 °C") or to a range (e.g. "about x to approximately y"), the value or range is interpreted as being as accurate as the method used to measure it. Unless explicitly stated otherwise, the general convention in the scientific and technical literature may be applied so that the last digit of numerical values preferably indicates the precision of measurement. Thus, unless other error margins are given, the maximum margin is preferably ascertained by applying the rounding-off convention to the last decimal place. For instance, a value of 3.5 preferably has an error margin of 3.45 to 3.54 and a range of 2% to 10% preferably covers a range of 1.5% to 10.4%. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 % to about 5 %" should be interpreted to include not only the explicitly recited values of about 1 % to about 5 %, but also include individual values and sub-ranges within the indicated range. Thus, included in this numerical range are individual values such as 2, 3, and 4 and sub-ranges such as from 1-3, from 2-4, and from 3-5, etc. This same principle applies to ranges reciting only one numerical value.

By "room temperature" or its abbreviation "rt" is meant herein that the reactions or processes are performed without heating or cooling. Generally, by room temperature may be understood as a temperature between about 15 °C and about 30 °C, or more particularly between about 20 °C and about 25 °C.

As used herein, the term "Lewis acid" refers to a molecule which can accept a pair of electrons and form a coordinate covalent bond.

As used herein, the term "aqueous solution of sulfuric acid" refers to dilute sulfuric acid, i.e. not concentrated. Preferably, it refers to an aqueous solution containing sulfuric acid in a maximum concentration of 90 % (w/w) (i.e. 90 gram of acid per 100 grams of solution), more preferably in a concentration range 10 - 90 % (w/w). An aqueous solution of sulfuric acid may be obtained by slowly adding concentrated sulfuric acid to water under stirring in a cool bath and monitoring the temperature of the solution to not increase excessively. The concentration of concentrated sulfuric acid is typically 95-98 % (w/w).

The term "organic solvent" includes for example cyclic and acyclic ethers (e.g. Et₂O, iPr₂O, tBu₂O, MeOtBu, 1,4-dioxane, tetrahydrofuran, methyltetrahydrofuran), hydrocarbon solvents (e.g. pentane, hexane, cyclohexane, heptane), halogenated solvents (e.g. dichloromethane, chloroform), alcohols (e.g. methanol, ethanol, propanol, isopropanol, sec-butanol, t-butanol), aromatic solvents (e.g. toluene, xylene), ketones (e.g. acetone, butanone, pentanone, methyl ethyl ketone, ethyl isopropyl ketone), esters (e.g. EtOAc, iPrOAc), nitriles (e.g. acetonitrile, benzonitrile, propionitrile), amides (e.g. DMF, DMA, HMPA), sulfoxides (DMSO) and mixtures thereof.

The term "vol" refers to volume equivalents, that is, the milliliters of solvent per gram reference starting material. For instance, 1 vol means 1 mL solvent per 1 g reference starting material.

The term "amino protecting group" (APG) refers to a group blocking the NH function for subsequent reactions that can be removed under controlled conditions. Amino protecting groups are well known in the art. Illustrative examples of amino protecting groups have been described by Green TW et al. in "Protective Groups in Organic Synthesis", 3rd Edition (1999), Ed. John Wiley & Sons. Virtually any amino protecting group can be used to put the invention into practice. Illustrative, non-limiting examples of APGs include:
- carbamates [-COOR]. R can be selected from C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl, 3- to 10-membered heteroaryl. Examples of carbamates include methyl carbamate (MOC), t-butyl carbamate (BOC), benzyl carbamate (CBz), 9-fluorenilmetil carbamate (FMOC), trichloroethyl carbamate (TROC), trimethylsilyl carbamate;
- amides [-COR]. R can be selected from C₁-C₆ alkyl, C₂-C₆ alkenyl, C₃-C₇ cycloalkyl, C₆-C₁₀ aryl, (C₆-C₁₀)aryl(C₁-C₆)alkyl, 3- to 10-membered heterocyclyl, 3- to 10-membered heteroaryl. Examples of amides include formamide, acetamide, phenylacetamide, haloacetamide, benzamide, picolinamide;
- amines [-R]. R can be selected from C₁-C₆ alkyl, C₆-C₁₀ aryl and (C₆-C₁₀)aryl(C₁-C₆)alkyl. Examples of amines include methyl amine, tert-butyl amine, benzyl amine, p-methoxybenzyl amine, 3,4-dimethoxybenzyl amine, allyl amine, methoxymethyl amine, triphenylmethyl amine, benzoyl amine, dinitrophenyl amine, p-methoxyphenyl amine; and
- silyl amines [-Si(R)(R')(R")]. R, R' and R" can be independently selected from C₁-C₆ alkyl, C₃-C₇ cycloalkyl, C₆-C₁₀ aryl, C₁-C₆ alkoxy and halogen. Examples of silyl amines include trimethylsilyl amine, triethylsilyl amine, tert-butyldimethylsilyl amine, tert-butyldiphenylsilyl amine, tri-isopropylsilyl amine, triphenylsilyl amine.

As the skilled person knows, amino protecting groups are commonly named considering the nitrogen atom. Thus, terms such as "carbamate", "amide", "amine", and "silyl amine" really refer herein to the chemical group formed with the nitrogen atom i.e. NCOOR, NCOR, NR, or NSi(R)(R')(R"), respectively.

The term "alkyl" refers to a linear or branched alkane derivative containing from 1 to 6 ("C₁-C₆ alkyl"), preferably from 1 to 3 ("C₁-C₃ alkyl"), carbon atoms and which is bound to the rest of the molecule through a single bond. Illustrative examples of alkyl groups include methyl, ethyl, n-propyl, i-propyl, n-butyl, i-butyl, t-butyl, pentyl, hexyl.

The term "alkenyl" refers to a linear or branched hydrocarbon chain radical containing from 2 to 6 ("C₂-C₆ alkenyl"), preferably from 2 to 3 ("C₂-C₃ alkenyl"), carbon atoms and which contains at least one double bond and is attached to the rest of the molecule by a single bond. Examples of alkenyl groups include ethenyl, propenyl, allyl, butenyl, 1-methyl-2-buten-1-yl, and the like.

The term "cycloalkyl" refers to a radical derived from cycloalkane containing from 3 to 7 ("C₃-C₇ cycloalkyl"), preferably from 3 to 6 ("C₃-C₆ cycloalkyl") carbon atoms. Illustrative examples of cycloalkyl groups include cyclopropyl, cyclobutyl, cyclopentyl, cyclohexyl, etc.

The term "aryl" refers to an aromatic group having between 6 and 10, preferably 6 or 10 carbon atoms, comprising 1 or 2 aromatic nuclei, bound by means of a carbon-carbon bond or fused. Illustrative examples of aryl groups include phenyl, naphthyl, diphenyl, indenyl, etc. Preferably, it is phenyl.

The term "arylalkyl" refers to an alkyl group as defined above substituted with an aryl group as defined above, such as e.g. (C₆-C₁₀)aryl(C₁-C₆)alkyl and (C₆-C₁₀)aryl(C₁-C₃)alkyl. Examples of such groups include benzyl, phenylethyl, phenylpropyl, naphthylmethyl, etc. Preferably, it is benzyl.

The term "heterocyclyl" refers to a saturated or partially unsaturated monocyclic or bicyclic system containing from 3 to 10, preferably 5 to 7, ring atoms containing one or more, specifically one, two, three or four ring heteroatoms independently selected from N, O, and S, and the remaining ring atoms being carbon. Illustrative examples of heterocyclyl groups include tetrahydropyran, morpholine, piperazine, piperidine and [1,4]dioxane.

The term "heteroaryl" refers to an aromatic monocyclic or bicyclic system containing from 3 to 10, preferably 5 to 7, ring atoms containing one or more, specifically one, two, three or four ring heteroatoms independently selected from O, N and S, and the remaining ring atoms being carbon. Illustrative examples of heteroaryl groups include pyrrole, furan, thiophene, pyridine and pyrimidine.

The term "alkoxy" designates an alkyl group as defined above having between 1 and 6 carbon atoms ("C₁-C₆ alkoxy"), preferably between 1 and 3 carbon atoms ("C₁-C₃ alkoxy"), linked to the rest of the molecule through oxygen. Examples of alkoxy include methoxy, ethoxy, isopropoxy, tertbutoxy, and the like.

The term "halogen" refers to bromine, chlorine, iodine or fluorine.

As understood in this technical area, there may be a certain degree of substitution in the aforementioned radicals. Therefore, there may be substitution in any of the groups of the present invention. The previous groups can be substituted in one or more available positions with one or more substituents. Said substituents include, for example and in non-limiting sense, C₁₋₆ alkyl, C3-7 cycloalkyl, C₆-C₁₀ aryl, 3- to 10-membered heterocyclyl, 3- to 10-membered heteroaryl, halogen, -CN, NO₂, CF₃, -N(Rₐ)(R_{b}), -OR_{c}, -SR_{d}, -C(O)Rₑ, -C(O)OR_{f}, -C(O)N(R_{g})(Rₕ), -OC(O)Rᵢ; wherein Rₐ, R_{b}, R_{c}, R_{d}, Rₑ, R_{f}, R_{g}, Rₕ and Rᵢ are independently selected from hydrogen, C₁-C₆ alkyl, C₆-C₁₀ aryl, 3- to 10-membered heterocyclyl, 3- to 10-membered heteroaryl and trifluoromethyl.

The invention also provides "salts" of the compounds described in the present description. By way of illustration, said salts can be acid addition salts, base addition salts or metal salts, and can be synthesized from the parent compounds containing a basic or acid moiety by means of conventional chemical processes known in the art. Such salts are generally prepared, for example, by reacting the free acid or base forms of said compounds with a stoichiometric amount of the suitable base or acid in water or in an organic solvent or in a mixture of the two. Non-aqueous media such as ether, ethyl acetate, ethanol, acetone, isopropanol or acetonitrile are generally preferred. Illustrative examples of said acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, p-toluenesulfonate, trifluoroacetate, camphorsulfonate, etc. Illustrative examples of base addition salts include inorganic base salts such as, for example, ammonium salts and organic base salts such as, for example, ethylenediamine, ethanolamine, *N,N*-dialkylenethanolamine, triethanolamine, glutamine, amino acid basic salts, etc. Illustrative examples of metal salts include, for example, sodium, potassium, calcium, magnesium, aluminum and lithium salts.

In a particular embodiment, the salt is an acid addition salt, such as hydrochloride, hydrobromide, hydroiodide, sulfate, nitrate, phosphate, acetate, maleate, fumarate, citrate, oxalate, succinate, tartrate, malate, mandelate, methanesulfonate, p-toluenesulfonate, trifluoroacetate or camphorsulfonate. Preferably, it is selected from HCI, H₂SO₄, pTsOH, oxalate and fumarate salt.

The term "solvate" according to this invention is to be understood as meaning any form of the compound which has another molecule (most likely a polar solvent) attached to it via non-covalent bonding. Examples of solvate include hydrates and alcoholates, e.g. methanolates. Methods of solvation are generally known within the art. The compounds of the invention may present different polymorphic forms, and it is intended that the invention encompasses all such forms.

### Preparation of compounds of general formula (III)

The compounds of general formula (III) and salts or solvates thereof can be obtained by a process comprising two steps, as depicted in the following scheme:

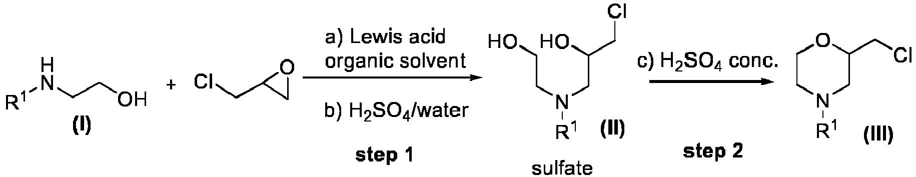

### Step 1

The first part (a) of step 1 involves the reaction between epichlorohydrin with a compound of general formula (I) or a salt or solvate in the presence of a Lewis acid and an organic solvent to obtain a compound of general formula (II) or a salt or solvate thereof.

R¹ in the compounds of general formula (I), (II) and (III) is an amino protecting group, which is preferably selected from a carbamate, an amide, an amine or a silyl amine, as previously defined. In a more particular embodiment, the amino protecting group is a benzyl amine, i.e. R¹ is -CH₂-Ph.

The amount of epichlorohydrin may typically range from 1 to 2 equivalents or from 1 to 1.5 equivalents (e.g. about 1.0, 1.1, 1.2, 1.3, 1.4 or 1.5 eq.) with respect to the compound of general formula (I). Larger amounts may be used but are generally unnecessary. In a particular embodiment, about 1.1 or 1.2 eq. of epichlorhydrin are used.

Suitable Lewis acids include, but are not limited to, LiClO₄, LiCI, LiBr, Lil·2H₂O, CsClO₄, Mg(ClO₄)₂, MgCl₂, MgBr₂, Mg(OAc)₂, CaCl₂, CaSO₄, TiCl₄, FeCl, FeCl₂, FeCl₃, CuCI, CuBr, CuCl₂, CuBr₂, ZnCl₂, ZnBr₂, Zn(OAc)₂, BBr₃, BF₃, BF₃·OEt₂, B(OPh)₃, B(OCH₃)₃, SnCI, TiCI, AlCl₃, SnCl₂, SnBr₂, SnCl₄, and SnBr₄ or a mixture thereof. In a particular embodiment, the Lewis acid is selected from the group consisting of LiClO₄, LiCI, LiBr, Lil·2H₂O,CsClO₄, and Mg(ClO₄)₂ or a mixture thereof, and more particularly, LiClO₄.

The amount of Lewis acid (e.g. LiClO₄) may typically range from 1 to 2 equivalents or from 1 to 1.5 equivalents (e.g. about 1.0, 1.1, 1.2, 1.3, 1.4 or 1.5 eq.) with respect to the compound of general formula (I). Larger amounts may be used but are generally unnecessary. In a particular embodiment, about 1.1 or 1.2 eq. of Lewis acid are used.

The reaction between epichlorohydrin and a compound of general formula (I) or a salt or solvate is carried out in the presence of an organic solvent, such as for example a cyclic or acyclic ether (e.g. Et₂O,iPr₂O, tBu₂O, MeOtBu, 1,4-dioxane, tetrahydrofuran, methyltetrahydrofuran), a hydrocarbon solvent (e.g. pentane, hexane, cyclohexane, heptane), a halogenated solvent (e.g. dichloromethane, chloroform), an aromatic solvent (e.g. toluene, xylene), a ketone (e.g. acetone, butanone, pentanone, methyl ethyl ketone, ethyl isopropyl ketone), a sulfoxide (e.g. dimethylsulfoxide) or mixtures thereof. In a particular embodiment, the reaction is performed in the presence of an aromatic solvent, such as toluene and/or xylene, preferably toluene.

The amount of the organic solvent (e.g. toluene or xylene) may typically range from 5 vol to 20 vol (e.g. about 5, 6, 7, 8, 9, 10, 11, 12, 13, 14, 15, 16, 17, 18, 19 or 20 vol), and more particularly from 10 vol to 16 vol. In a particular embodiment, about 10, 11, 12, 13, 14, 15 or 16 vol of organic solvent are used.

The reaction may be carried out at a temperature between 0 °C and 50 °C, more particularly between rt and 40 °C, and even more particularly at about 35 °C.

In a preferred embodiment, about 1 eq. of compound of general formula (I) or a salt or solvate, about 1.2 eq. of epichlorohydrin and about 1.1 eq. of LiClO₄ are placed in about 10 Vol of toluene and the reaction takes place for between 2-4 h at about 35 °C.

The second part (b) of step 1 involves the work-up of the reaction mixture. Once the reaction is complete, the reaction mixture may be worked-up by a process comprising mixing the reaction mixture with an aqueous solution of sulfuric acid to obtain a water-sulfuric phase comprising a sulfate salt of the compound of general formula (II) and an organic phase. Non-basic impurities remain in the organic phase and can therefore be easily removed.

Preferably, before mixing with an aqueous solution of sulfuric acid, the reaction mixture is washed with water and/or NaCl aqueous solution in order to remove the Lewis acid.

The aqueous solution of sulfuric acid normally has a concentration not higher than 90 % (w/w), e.g. 10 - 90 % (w/w), 20 - 80 % (w/w), 30 - 70 % (w/w), or 40 - 60 % (w/w). In a particular embodiment, H₂SO₄ 50% w/w is used.

A particular working-up process of the reaction mixture comprises: one or more water washings and/or one or more washings with NaCl aqueous solution and mixing the remaining organic phase with an aqueous solution of sulfuric acid so that the corresponding intermediate compound of general formula (II) is extracted as a sulfate salt from the organic phase into the water-sulfuric phase that can be used directly, i.e. without isolation, in the second step.

In a more particular embodiment, the working-up process comprises washing the reaction mixture with water (e.g. about 10 vol or more) so as to obtain an aqueous phase and an organic phase, separating the aqueous and the organic phase, washing the organic phase with NaCl aqueous solution (e.g. about 10 vol or more of 12 % NaCl aqueous solution) so as to obtain a second aqueous phase and a second organic phase, separating the second aqueous phase and the second organic phase, and mixing an aqueous solution of sulfuric acid (e.g. about 1-2 vol of 50% w/w H₂SO₄ aq) with the second organic phase. The water-sulfuric phase, which contains the intermediate compound of general formula (II) as a sulfate salt, may be then separated and subjected to further transformation to afford a compound of general formula (III) or a salt or solvate thereof.

More particular preferred conditions for the working-up process are:
- washing the reaction mixture with about 10 vol H₂O so as to obtain an aqueous phase and an organic phase;
- separating the aqueous and the organic phase;
- washing the organic phase with about 10 vol NaCl 12% w/w so as to obtain a second aqueous phase and a second organic phase;
- separating the second aqueous and the second organic phase; and
- extracting the second organic phase with about 1.55 vol H₂SO₄ aq 50% w/w (about 1.66 eq with respect to starting compound of general formula (I)) so as to obtain a water-sulfuric phase comprising a sulfate salt of the compound of general formula (II)).

### Step 2

The second step involves increasing the concentration of sulfuric acid by mixing the water-sulfuric phase comprising a sulfate salt of the compound of general formula (II) obtained in step 1 with concentrated sulfuric acid such as H₂SO₄ 95-98% (w/w) to obtain the compound of general formula (III) or a salt or solvate thereof.

Concentrated sulfuric acid may be typically added until reaching from 5 to 15 equivalents (e.g. about 5, 6, 7, 8, 9, 10, 11, 12, 13, 14 or 15 eq) of sulfuric acid in the reaction mixture, preferably until reaching about 10 equivalents (about 86% w/w H₂SO₄ aq) with respect to the compound of general formula (I). Thus, considering that the water-sulfuric phase containing the sulfate salt of a compound of general formula (II) obtained from step 1 already contains sulfuric acid, the amount of concentrated sulfuric acid added or mixed in step 2 may typically range from 1 to 5 vol (e.g. about 1, 2, 3, 4 or 5 vol), preferably about 3 vol (about 8.5 eq), with respect to the compound of general formula (I).

In a preferred embodiment, 3 vol H₂SO₄ 98% w/w (about 8.5 eq with respect to the starting compound of general formula (I)) are mixed with the water-sulfuric phase comprising a sulfate salt of the compound of general formula (II) obtained in step 1.

Then, the reaction mixture is normally heated at 100 °C - 140 °C (e.g. about 120 °C - 125 °C). Typically, the reaction takes place in 5 h - 16 h, e.g. about 8 or 9 h.

Once the reaction is complete, the reaction mixture may be worked-up by a process comprising: adding water, dichloromethane (DCM) and a base such as NaOH or NH₃ to the reaction mixture, separating an aqueous and an organic phase and distilling or partially distilling the organic phase. Temperature control during the additions of water and base is very advisable for safety reasons, especially when performing large scale synthesis.

More particular preferred conditions for the working-up process are:
- adding about 20 vol H₂O, about 10 vol DCM, and about 11 vol NH₃ aq 25% w/w to the reaction mixture so as to obtain an aqueous phase and an organic phase;
- separating the aqueous and the organic phase; and
- concentrating the organic phase comprising the compound of general formula (III).

The compound of general formula (III) can be isolated, for instance in salt form.

These salts can be obtained by means of conventional methods by reacting the compound of general formula (III) with acid in a solvent in which the salt is insoluble so that it directly precipitates and can be separated, e.g. by filtration. Precipitation may be favoured by cooling down the reaction mixture and/or partially distilling the solvent.

Alternatively, the compound of general formula (III) and may be used directly, i.e. without isolation, in the subsequent step (conversion into compounds of formula (IV)). In such a case, more particular preferred conditions for the working-up process are:
- adding about 20 vol H₂O, about 10 vol DCM, and about 11 vol NH₃ aq 25% w/w to the reaction mixture so as to obtain an aqueous phase and an organic phase;
- separating the aqueous and the organic phase;
- concentrating the organic phase to about 3 vol;
- adding about 3 vol toluene and concentrating to 3 vol twice so as to obtain 3 vol organic phase (e.g. toluene).

The resulting organic phase from the working-up process comprises the compound of general formula (III) and may be used directly in the subsequent conversion into compounds of formula (IV) (one-pot approach).

### Particular salts of compounds of formula (III)

The invention is also directed to certain salts of 4-benzyl-2-(chloromethyl)morpholine, i.e. the compound of general formula (III) wherein R¹ is benzyl. Specifically, the invention encompasses the oxalate, the fumarate and the tosylate salt of 4-benzyl-2-(chloromethyl)morpholine, the structures of which are as follows.

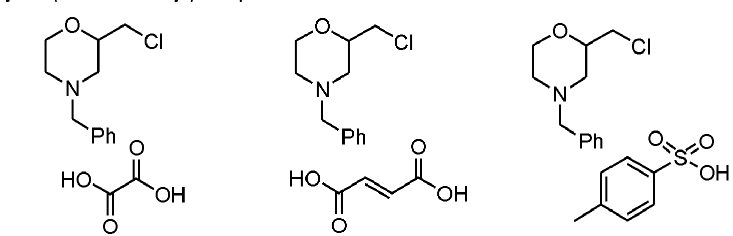

These salts can be obtained by means of conventional methods by reacting 4-benzyl-2-(chloromethyl)morpholine with the corresponding acid, namely oxalic acid, fumaric acid or p-toluenesulfonic acid. In an embodiment, the formation of these acid addition salts is performed in a solvent in which the salt is insoluble so that it directly precipitates and can be separated, e.g. by filtration. Precipitation may be favoured by cooling down the reaction mixture and/or partially distilling the solvent.

For instance, 4-benzyl-2-(chloromethyl)morpholine oxalate can be obtained by reacting 4-benzyl-2-(chloromethyl)morpholine with oxalic acid in a suitable organic solvent, such as a mixture of acetone/ethanol/heptane, so that said salt precipitates, facilitating its isolation.

In a particular embodiment, to a solution of 4-Benzyl-2-(chloromethyl)morpholine (3) in acetone (e.g. about 5 Vol), a solution of oxalic acid (e.g. about 1 eq) in ethanol (e.g. about 2 Vol) is added. Heptane (e.g. about 5 vol) is then added and the resulting slurry is filtered, washed with heptane and dried, giving rise to the oxalate salt of 4-Benzyl-2-(chloromethyl)morpholine.

For instance, 4-benzyl-2-(chloromethyl)morpholine fumarate can be obtained by reacting 4-benzyl-2-(chloromethyl)morpholine with fumaric acid in a suitable organic solvent, such as isopropanol or isopropanol/heptane, so that said salt precipitates, facilitating its isolation.

In a particular embodiment, a solution of 4-Benzyl-2-(chloromethyl)morpholine (3) in isopropanol (e.g. about 3 Vol) is added to a solution of fumaric acid (e.g. about 1.2 eq) in isopropanol (e.g. about 8 Vol) and the mixture is heated, preferably at about 70°C. The mixture is cooled down to room temperature, and afterwards, some solvent is vacuum distilled until some crystals appear (e.g. about 3-4 vol). Preferably, once the precipitate is formed, heptane is added (e.g. about 5 vol) to facilitate stirring. The suspension formed is filtered, rinsed with heptane and dried, giving rise to the fumarate salt of 4-Benzyl-2-(chloromethyl)morpholine.

For instance, 4-benzyl-2-(chloromethyl)morpholine tosylate can be obtained by reacting 4-benzyl-2-(chloromethyl)morpholine with p-toluenesulfonic acid in a suitable organic solvent, such as a mixture of acetone and ethanol, so that said salt precipitates, facilitating its isolation.

In a particular embodiment, to a solution of 4-Benzyl-2-(chloromethyl)morpholine (**3**) in acetone (e.g. about 10 Vol) at room temperature, a solution of p-toluenesulfonic acid (e.g. about 1 eq) in EtOH (e.g. about 1 Vol) is added slowly, and a precipitate is formed. The suspension formed is filtered, rinsed with acetone and dried, giving rise to the tosylate salt of 4-Benzyl-2-(chloromethyl)morpholine.

The above-mentioned salts of 4-benzyl-2-(chloromethyl)morpholine are new compounds, can be used in the synthesis of viloxazine or a salt or solvate thereof, and therefore constitute an additional aspect of this invention as does their use in obtaining viloxazine or a salt or solvate thereof. The process for obtaining said salts of 4-benzyl-2-(chloromethyl)morpholine constitutes a further aspect of this invention.

### Conversion from (III) into (IV)

The compounds of general formula (IV) and salts or solvates thereof can be obtained from the compounds of general formula (III) and salts or solvates thereof by reaction with 2-ethoxyphenol in an organic solvent in the presence of a base, as depicted in the following scheme:

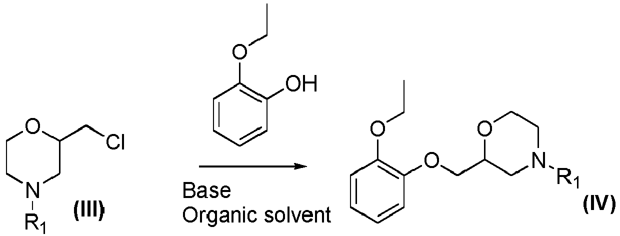

In a particular embodiment, the compound of general formula (III) is used as a free base. In another particular embodiment, the compound of general formula (III) is used as a salt such as the oxalate, the fumarate or the tosylate salt of 4-benzyl-2-(chloromethyl)morpholine. If the compound of general formula (III) is in the form of a salt such as an oxalate salt, the free form of the compound of general formula (III) can be initially obtained by treating the salt with base (e.g. extraction with NaHCO₃).

In a particular embodiment, the compound of general formula (III) or a salt or solvate thereof is used without isolation for obtaining a compounds of general formula (IV) or a salt or solvate thereof (one-pot process).

The amount of 2-ethoxyphenol may typically range from 1 to 6 equivalents (e.g. about 1.0, 1.5, 2.0, 2.5, 3.0, 3.5, 4.0, 4.5, 5.0, 5.5 or 6.0 eq.) with respect to the compound of general formula (I). Larger amounts may be used but are generally unnecessary. In a particular embodiment, the amount of 2-ethoxyphenol ranges from 1 to 1.5 equivalents (e.g. about 1.0, 1.1, 1.2, 1.3, 1.4 or 1.5 eq.) with respect to the compound of general formula (I). In a more particular embodiment, about 1 or 1.1 of 2-ethoxyphenol are used.

Suitable bases include hydroxides, alkali metal hydrides and alkali metal alcoholates, such as e.g. NaOH, KOH, NaH, NaOtBu, KOtBu, NaOMe, NaOEt. In a particular embodiment, the base is KOH.

The amount of base (e.g. KOH) may typically range from 1 to 6 equivalents (e.g. about 1.0, 1.5, 2.0, 2.5, 3.0, 3.5, 4.0, 4.5, 5.0. 5.5 or 6.0 eq.) with respect to the compound of general formula (I). Larger amounts may be used but are generally unnecessary. In a particular embodiment, the amount of base ranges from 1 to 1.5 equivalents (e.g. about 1.0, 1.1, 1.2, 1.3, 1.4 or 1.5 eq.) with respect to the compound of general formula (I). In a more particular embodiment, about 1 or 1.1 eq of base are used.

The reaction between 2-ethoxyphenol and a compound of general formula (III) or a salt or solvate may be conveniently carried out in the presence of an organic solvent, such as for example a cyclic or acyclic ether (e.g. Et₂O,iPr₂O, tBu₂O, MeOtBu, 1,4-dioxane, tetrahydrofuran, methyltetrahydrofuran), a hydrocarbon solvent (e.g. pentane, hexane, cyclohexane, heptane), a halogenated solvent (e.g. dichloromethane, chloroform), an alcohol (e.g. methanol, ethanol, propanol, isopropanol, sec-butanol, t-butanol), an aromatic solvent (e.g. toluene, xylene), a ketone (e.g. acetone, butanone, pentanone, methyl ethyl ketone, ethyl isopropyl ketone), an ester (e.g. ethyl acetate, isopropyl acetate), a nitrile (e.g. acetonitrile, benzonitrile, propionitrile), an amide (e.g. dimethylformamide, dimethylacetamide, hexamethylphosphoramide), a sulfoxide (e.g. dimethylsulfoxide) or mixtures thereof. In a particular embodiment, the reaction is performed in the presence of an amide solvent such as DMF, a sulfoxide solvent such as DMSO, or in the presence of a mixture of a sulfoxide solvent and an aromatic solvent, such as mixtures of DMSO and toluene.

If the compound of general formula (III) or a salt or solvate thereof is used without isolation (one-pot process), it was already comprised within an organic phase, e.g. toluene. In such a case, the addition of DMSO will result in an organic solvent consisting in a mixture of DMSO and toluene.

The amount of the organic solvent (e.g. DMF, DMSO or DMSO/Tol) for the conversion into a compound of general formula (IV) or a salt or solvate thereof may typically range from 5 vol to 20 vol (e.g. about 5, 6, 7, 8, 9, 10, 11, 12, 13, 14, 15, 16, 17, 18, 19 or 20 vol), and more particularly 11-13 vol. In a particular embodiment, the organic solvent is 11 vol DMF. In another particular embodiment, the organic solvent is a mixture of 3 vol toluene (coming from the previous step) and 10 vol DMSO.

The reaction may be carried out at a temperature between 80 °C and 150 °C, more particularly between 100 °C and 120 °C, and even more particularly at about 100 °C or 110 °C.

In a preferred embodiment, the compound of general formula (III) or a salt or solvate thereof obtained from about 1 eq. of compound of general formula (I) or a salt or solvate reacts with about 4 eq. of 2-ethoxyphenol and about 4 eq. of KOH in about 11 Vol of DMF and the reaction takes place for about 2h at about 110 °C.

In a preferred embodiment, the compound of general formula (III) or a salt or solvate thereof obtained from about 1 eq. of compound of general formula (I) or a salt or solvate reacts with about 1.1 eq. of 2-ethoxyphenol and about 1.1 eq. of KOH in about 13 Vol of DMSO or 13 Vol DMSO/toluene (10 vol DMSO + 3 vol toluene) and the reaction takes place for about 2 h at about 100 °C.

Once the reaction is complete, the reaction mixture may be worked-up by a process comprising: adding water and extracting with an organic solvent such as ethyl acetate or toluene. Temperature control during the addition of water is very advisable for safety reasons, especially when performing large scale synthesis.

More particular preferred conditions for the working-up process are:
- adding about 20 vol H₂O and about 10 vol ethyl acetate to the reaction mixture to obtain and organic and an aqueous phase;
- separating the organic phase and the aqueous phase;
- extracting the aqueous phase with about 3 vol ethyl acetate to obtain a second organic phase;
- combining the two organic phases:
- washing the combined organic phases with water; and
- concentrating the combined organic phases, e.g. until about 10 vol.

More particular preferred conditions for the working-up process are:
- adding about 10 vol H₂O and about 1 vol toluene to the reaction mixture to obtain and organic and an aqueous phase;
- separating the organic phase and the aqueous phase;
- extracting the aqueous phase with about 2 vol toluene to obtain a second organic phase;
- combining the two organic phases; and
- distilling the combined organic phases. e.g. until about 3 or 4 vol (anhydration).

The obtained products of formula (IV) generally do not require subsequent purification but, if desired, they can additionally be purified by means of conventional and industrially acceptable processes, such as for example by means of a crystallization process, either of the product as a free base (in the event that it is a solid) or, more preferably, through the formation of an addition salt (in the event that it is an oil). Illustrative, non-limiting examples of suitable solvents for said crystallization include ketones (e.g., acetone, etc.), nitriles (e.g., acetonitrile, etc.), alcohols (e.g., i-PrOH, EtOH, etc.), esters (e.g., ethyl acetate (AcOEt), etc.), ethers, etc., preferably acetone or acetonitrile.

Illustrative, non-limiting examples of suitable acid addition salts include inorganic acid addition salts such as, for example, hydrochloride, hydrobromide, hydroiodide, sulfate, nitrate, phosphate, etc.; and organic acid addition salts such as, for example, acetate, maleate, fumarate, citrate, oxalate, succinate, tartrate, malate, mandelate, methanesulfonate, p-toluenesulfonate, trifluoroacetate, camphorsulfonate etc. In an embodiment, the obtained product of formula (IV) is transformed into its oxalate salt for purification purposes.

For instance, the oxalate salt of a compound of general formula (IV) may be obtained by a process comprising: adding a solution of oxalic acid to the worked-up reaction mixture to form a suspension, adding heptane to the suspension, stirring the suspension, filtering the suspension, washing with heptane and drying the solid formed.

More particular preferred conditions for purification by forming and crystallizing the the oxalate salt of a compound of general formula (IV) are:
- adding about 6 or 7 vol toluene to the compound of general formula (IV) in about 3 or 4 vol toluene (about 10 vol toluene in total);
- adding about 2 vol heptane;
- adding solution of oxalic acid, said solution having been obtained by dissolving about 1 eq of oxalic acid with respect to the initial compound of general formula (I) in about 3 vol ethanol, to obtain a suspension;
- filtering the suspension to obtain a solid; and
- washing the solid with about 2 vol toluene and about 2 vol heptane.

### Conversion from (IV) into (V)

The compound of formula (V) and salts or solvates thereof can be obtained from the compounds of general formula (IV) and salts or solvates thereof by reaction with ethyl chloroformate, as depicted in the following scheme:

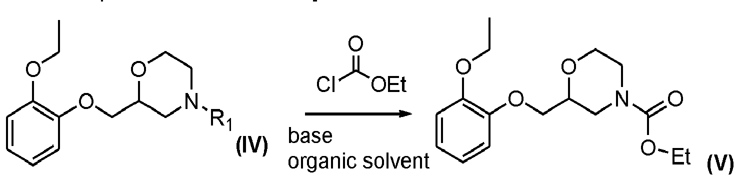

Preferably, R¹ is a Benzyl group (Bn). If R¹ is not Bn, other conditions could be employed for removal of the amino protecting group, as the skilled person clearly recognizes.

In a particular embodiment, the compound of general formula (IV) is used as a free base. In another particular embodiment, the compound of general formula (IV) is used as a salt. If the compound of general formula (IV) is in the form of a salt such as an oxalate salt, the free form of the compound of general formula (IV) can be initially obtained by treating the salt with base (e.g. extraction with NaHCO₃).

The amount of ethyl chloroformate may typically range from 1.1 to 3 equivalents, more preferably from 1.5 to 2.5 equivalents (e.g. about 1.5, 1.6, 1.7, 1.8, 1.9, 2, 2.1, 2.2, 2.3, 2.4 or 2.5 eq.) with respect to the compound of general formula (I). Larger amounts may be used but are generally unnecessary. In a particular embodiment, about 2 eq. of ethyl chloroformate are used.

Suitable bases include an amine such as DIPEA. In a particular embodiment, the base is DIPEA.

The amount of base (e.g. DIPEA) may typically range from 1 to 2 equivalents (e.g. about 1.0 1.1, 1.2, 1.3, 1.4, 1.5, 1.6, 1.7, 1.8, 1.9 or 2.0 eq.) with respect to the compound of general formula (I). Larger amounts may be used but are generally unnecessary. In a particular embodiment, about 1 eq. of base is used.

The reaction between ethyl chloroformate and a compound of general formula (IV) or a salt or solvate is carried out in the presence of an organic solvent, such as for example a cyclic or acyclic ether (e.g. Et₂O, iPr₂O, tBu₂O, MeOtBu, 1,4-dioxane, tetrahydrofuran, methyltetrahydrofuran), a hydrocarbon solvent (e.g. pentane, hexane, cyclohexane, heptane), a halogenated solvent (e.g. dichloromethane, chloroform), an alcohol (e.g. methanol, ethanol, propanol, isopropanol, sec-butanol, t-butanol), an aromatic solvent (e.g. toluene, xylene), a ketone (e.g. acetone, butanone, pentanone, methyl ethyl ketone, ethyl isopropyl ketone), an ester (e.g. ethyl acetate, isopropyl acetate), a nitrile (e.g. acetonitrile, benzonitrile, propionitrile), an amide (e.g. dimethylformamide, dimethylacetamide, hexamethylphosphoramide), a sulfoxide (e.g. dimethylsulfoxide) or mixtures thereof. In a particular embodiment, the reaction is performed in the presence of an aromatic solvent such as toluene or an halogenated solvent such as DCM.

The amount of the organic solvent (e.g. toluene or DCM) may typically range from 3 vol to 10 vol (e.g. about 3, 4, 5, 6, 7, 8, 9 or 10 vol), and more particularly about 6 vol.

The reaction may be carried out at a temperature of between 40 °C and 50 °C, i.e. at reflux temperature when DCM is the solvent used.

In a preferred embodiment, the compound of general formula (IV) or a salt or solvate thereof obtained from about 1 eq. compound of general formula (I) or a salt or solvate reacts with about 1.5 - 2 eq. of ethyl chloroformate and about 1 eq. DIPEA in about 6 Vol of toluene or DCM and the reaction takes place for between 2-4 h at about 40 °C - 50 °C.

Once the reaction is complete, the reaction mixture may be worked-up by a process comprising:
- adding water to the reaction mixture, or if needed, hydrolysis of the excess of ethyl chloroformate with water and base to obtain an organic and an aqueous phase;.
- separating an aqueous and an organic phase;
- extracting the aqueous phase with an organic solvent such as toluene;
- combining the organic phases; and
- concentrating.

More particular preferred conditions for the working-up process are:
- adding water to reaction mixture or hydrolysis of unreacted ethyl chloroformate by adding about 0.5 eq DIPEA with respect to the compound of general formula (I) and about 5 vol H₂O and heating the resulting mixture at reflux for about 1 h to obtain an organic and an aqueous phase;
- separating the organic and the aqueous phase;
- extracting the aqueous phase with 2 vol toluene or DCM to obtain a second organic phase;
- combining the two organic phases to obtain a combined organic phase; and
- distilling the combined organic phase until 2 vol (anhydration).

Afterwards, the reaction product may be purified, e.g. by a process comprising crystallization in heptane.

More particular preferred conditions for crystallization of the compound of general formula (V) and salts or solvates thereof are:
- adding about 12 vol heptane, optionally followed by cooling down the temperature (e.g. in an ice/water bath), so as to form a suspension; and
- filtering the suspension to obtain a solid and washing the solid formed with about 2 vol heptane.

More particular preferred conditions for crystallization of the compound of general formula (V) and salts or solvates thereof are:
- adding about 18 vol heptane and concentrating until 18 vol;
- adding about 3 vol heptane and concentrating until 18 vol, optionally followed by cooling down the temperature (e.g. in an ice/water bath), so as to form a suspension;
- filtering the suspension to obtain a solid and washing the solid formed with about 2 vol heptane twice.

### Conversion from (V) into Viloxazine

Viloxazine_and salts or solvates thereof can be obtained from the compounds of formula (V) and salts or solvates thereof by a process as depicted in the following scheme:

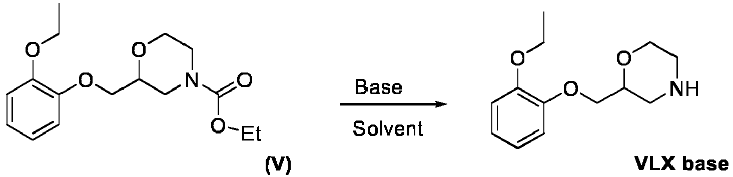

Suitable bases include hydroxides, alkali metal hydrides and alkali metal alcoholates, such as e.g. NaOH, KOH, NaH, NaOtBu, KOtBu, NaOMe, NaOEt. In a particular embodiment, the base is NaOH.

The amount of base (e.g. NaOH) may typically range from 1.5 to 4 equivalents (e.g. about 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9 or 4.0 eq.) with respect to the compound of general formula (I). Larger amounts may be used but are generally unnecessary. In a particular embodiment, about 3 eq of base are used.

The reaction between a base and a compound of formula (V) or a salt or solvate is carried out in the presence of an organic solvent, optionally along with water, such as for example a cyclic or acyclic ether (e.g. Et₂O, iPr₂O, tBu₂O, MeOtBu, 1,4-dioxane, tetrahydrofuran, methyltetrahydrofuran), a hydrocarbon solvent (e.g. pentane, hexane, cyclohexane, heptane), a halogenated solvent (e.g. dichloromethane, chloroform), an alcohol (e.g. methanol, ethanol, propanol, isopropanol, sec-butanol, t-butanol), an aromatic solvent (e.g. toluene, xylene), a ketone (e.g. acetone, butanone, pentanone, methyl ethyl ketone, ethyl isopropyl ketone), an ester (e.g. ethyl acetate, isopropyl acetate), a nitrile (e.g. acetonitrile, benzonitrile, propionitrile), an amide (e.g. dimethylformamide, dimethylacetamide, hexamethylphosphoramide), a sulfoxide (e.g. dimethylsulfoxide) or mixtures thereof. In a particular embodiment, the reaction is performed in the presence of an alcohol, such as isopropanol (IPA).

The amount of the organic solvent (e.g. IPA) may typically range from 5 vol to 15 vol (e.g. about 5, 6, 7, 8, 9, 10, 11, 12, 13, 14 or 15 vol), and more particularly about 9 vol. If used along with the organic solvent, the amount of water typically is 1 - 2 eq.

The reaction may be carried out at reflux temperature. In a particular embodiment, for instance when IPA or IPA/water is the solvent used, the temperature is between 80 °C and 85 °C.

In a preferred embodiment, the compound of formula (V) or a salt or solvate thereof obtained from about 1 eq. compound of general formula (I) or a salt or solvate reacts with about 3 eq. of NaOH in about 9 Vol of IPA (optionally, along with about 1 eq water) and the reaction takes place for about 16-18h at reflux.

Preferred conditions for the working-up process are:
- adding about 15 vol ethyl acetate and 10 vol water, preferably under temperature control for safety reasons so as to form an aqueous phase and an organic phase;
- extracting the aqueous phase with ethyl acetate;
- concentrating until about 11 vol;
- combining the organic phases; and
- concentrating the combined organic phase.

Preferred conditions for the working-up process are:
- adding about 10 vol water, preferably under temperature control for safety reasons;
- concentrating until about 11 vol;
- adding about 10 vol DCM to obtain an organic phase and an aqueous phase;
- separating the organic phase and the aqueous phase; and
- concentrating the organic phase until about 2 or 3 vol;

If desired, viloxazine free base may be further transformed in its hydrochloride salt by reaction with HCI.

Preferred conditions for the formation and crystallization of viloxazine HCI are:
- adding about 1.1 eq HCI in IPA (5-6N), optionally followed by cooling down the temperature, to form a suspension;
- filtering the suspension to form a solid; and
- washing the solid with about 2 vol AcOEt.

Preferred conditions for the formation and crystallization of viloxazine HCI are:
- adding about 14 vol AcOEt and concentrating until about 10 vol;
- adding about 1.1 eq HCI 37% w/w to form a suspension;
- filtering the suspension to form a solid; and
- washing the solid with about 3 vol AcOEt.

All the features described in this specification (including the claims, description and drawings) can be combined in any combination, with the exception of combinations of such mutually exclusive features.

The following examples are merely illustrative of certain embodiments of the invention and cannot be considered as restricting it in any way.

### EXAMPLES

### ¹H NMR

Proton Nuclear Magnetic Resonance analyses were recorded in deuterated chloroform (CDCl₃) dimethyl sulfoxide (DMSO-d6) in a 400 or 500 MHz spectrometer.

### DSC: differential scanning calorimetry

DSC analyses were recorded with a DSCQ100 (TA Instruments).

### XRPD: X-ray powder diffraction pattern

XRPD analysis was performed using a D8 DISCOVER DAVINCI (Bruker) diffractometer.

### Example 1. Formation of 4-Benzyl-2-(chloromethyl)morpholine (3) from N-Benzylethanolamine (1).

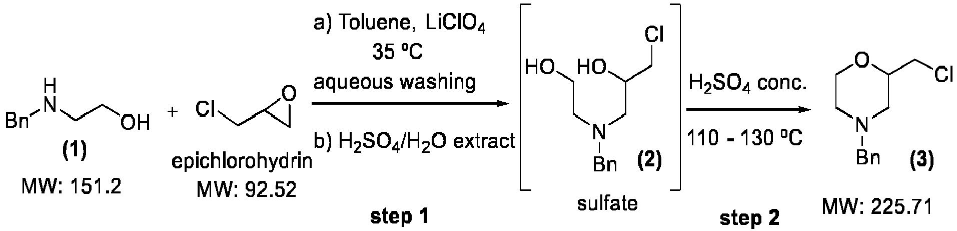

### Step 1: formation of intermediate (2) [A2VLX sulfate]

N-Benzylethanolamine (100 g, 0.66 mol) was dissolved in 1 L of toluene (10 vol), then 62 ml of epichlorhydrin (73.72 g, 1.2 eq) were added and stirring was started. 77.24 g of LiOCl₄ (1.1 eq) were finally added and the suspension or two phase system was warmed to 35 °C for 2.5 hours until the reaction was over.

The reaction mixture was cooled down to room temperature and 1 L of water (10 vol) was added. Then, the two-phase system formed was separated and the organic phase was washed again with 1 L of brine (12% w/w NaCl, 10 vol).

155 ml of a 50% w/w solution of aqueous sulfuric acid (1.66 eq of H₂SO₄) was added to the remaining toluene phase and the mixture was vigorously stirred. The intermediate (**2**) moved then to the aqueous phase, which was separated and subjected to step 2 without delay (storing of the water-sulfuric phase comprising the sulfate salt of intermediate (**2**) leads to the formation of impurities in just a few hours, even at low temperature).

### Step 2: formation of the morpholine (3) [A3VLX]

While stirring, 300 ml of sulfuric acid 98% (8.5 eq) were added to the aqueous phase mentioned above (final concentration of sulfuric acid: 86% w/w) and the reaction mixture was heated to 120 °C for 9 hours until the reaction was over.

The reaction mixture was cooled down to room temperature and 2 L of water (20 vol) were carefully added without exceeding the temperature of 30 °C. On this solution 1L of dichloromethane (10 vol) was added and the reaction mixture was neutralized with 1-1.2 L aqueous ammonia (25%, 10-12 vol), keeping the temperature below 30 °C at all times, until reaching a pH between 8-9.

The two phase system was separated, being the final product, 4-Benzyl-2-(chloromethyl)morpholine (3), in the organic phase.

As it will be discussed in example 3, the following three impurities are associated with the formation of the intermediate (2):

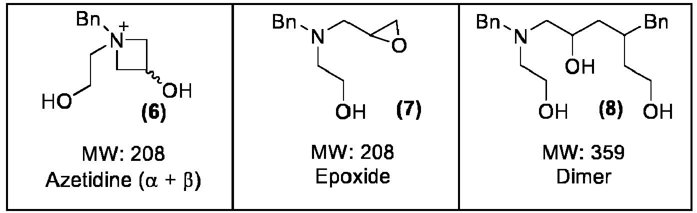

The dimer impurity (8) in turn may give rise to two additional impurities during the cyclization step with H₂SO₄ conc.

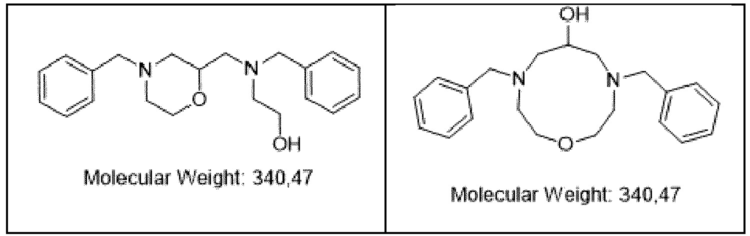

Advantageously, all these impurities are either not formed or formed in very small amounts in the process according to the present invention.

### Example 2. Formation of N-benzyl-2-(chloromethyl)morpholine (3) using prior art conditions (comparative example)

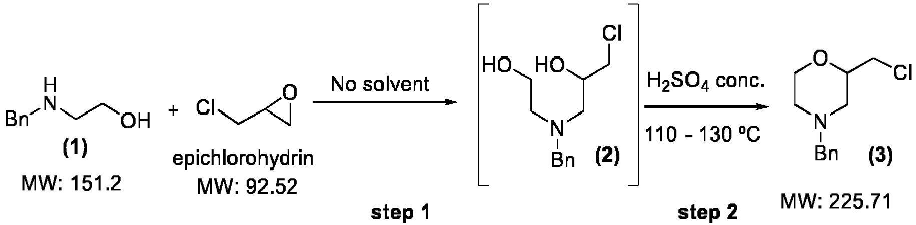

Racemic epichlorohydrin (6.2 ml, 1.2 eq) and benzylaminoethanol **(1)** (10 g, 8.58 ml, 66 mmol, 1 eq) were blended, a viscous solution was formed. The reaction mixture was stirred at room temperature.

Once the reaction was complete (2-5h), 25 ml of H₂SO₄ were added (strong exothermic behavior given rise to an increase of the temperature of the mixture until 70 °C*) and the mixture was heated until 130°C.

In one hour, the reaction mixture turned dark/black color and the reaction was finished. It was cooled down to room temperature and 100 ml of water were added keeping the system below room temperature.

Afterwards, 100 ml of dichloromethane were added and the mixture was basified with aqueous NaOH solution to basic medium.

The two layers were split and the organic phase was subjected to a vacuum distillation until residue.

Yield: 9 g/ 60% of a colored oil.

### Example 3 - Synthesis of intermediate (2) and impurity profile depending on the reaction conditions.

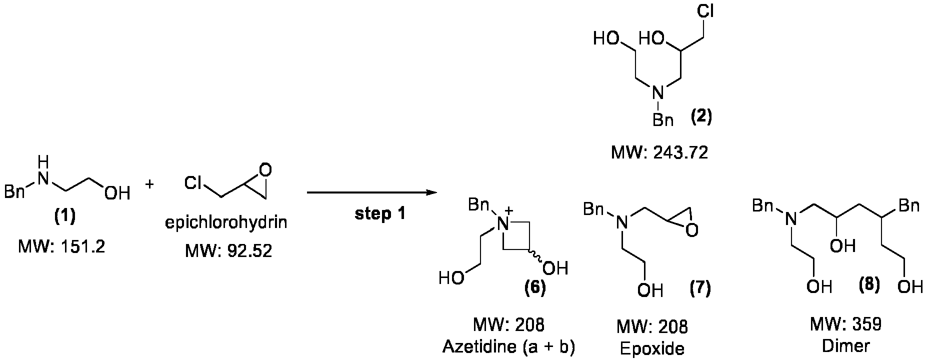

By means of basically the reaction conditions showed in examples 1 and 2 for the step 1, the impurity profile of the intermediate (2) obtained was assessed:

| Trial | Conditions | Impurity assessment | | |
|---|---|---|---|---|
| | | 5.5 h | 24 h | 48 h |
| **1** | 1 eq (**1**) | 1.5% SM (**1**) | 0% (**1**) | 0% (**1**) |
| | 1.2 eq epichlorohydrin | 1.6% azetidine (**6**) | 5.6% (**6**) | 11.1% (**6**) |
| | rt | 94% (FP) (**2**) | 6.7% epoxide (**7**) | 18.3% (**7**) |
| | Without solvent | | 85.2% (**2**) | 65% (**2**) |
| **2** | 1 eq **(1)** | 10% SM **(1)** | **0% (1)** | 0% **(1)** |
| | 0.9 eq epichlorohydrin | 1.3% dimer **(8)** | 3.9% **(6)** | 10.1% **(6)** |
| | rt | 85% FP **(2)** | 1.1% **(8)** | 7.2% **(8)** |
| | Without solvent | | 90% **(2)** | 76% **(2)** |
| **3** | 1 eq **(1)** | 2.85% SM **(1)** | **0% (1)** | |
| | 1.2 eq epichlorohydrin | 96.5% FP **(2)** | **0% (6)** | |
| | rt | | 0.3% epoxide **(7)** | |
| | Toluene (16 Vol) | | 0.2% dimer **(8)** | |
| | LiClO₄ 1.1 eq | | **99.2%** FP **(2)** | |

As it can be seen, intermediate (2) decomposes at room temperature in the reaction media, mainly to the azetidine, the epoxide and the dimer impurities. However, as compared with the prior art conditions (trials 1 and 2), the toluene/LiClO₄ conditions according to an embodiment of the present invention (trial 3) resulted in a higher yield of the desired compound (2) and less decomposition products and further the exothermic behavior was reduced during the subsequent step.

Intermediate (2) cannot be stored for a long time since it decomposes quite quickly. Further, if excess epichlorohydrin is removed by distillation under reduced pressure as proposed in the state of the art, the intermediate (2) also decomposes with the distillation step.

The only conditions that allow to keep the product stable in the reaction media are the use of organic solvent/Lewis acid system, where additionally the subsequent distillation is avoided by the use of H₂SO₄/water extractions.

### Example 4. Formation of N-benzyl-2-(chloromethyl)morpholine (3) according to prior art conditions (comparative example)

N-benzyl-2-(chloromethyl)morpholine (**3**) was prepared as disclosed by Loftus F, The Synthesis of Some 2-Substituted Morpholines, Synthetic Communications, 10(1), 59-73 (1980).

Specifically, N-Benzylethanolamine (5 g, 33 mmol) was dissolved in epichlorohydrin (25.8 ml, 330 mmol) and the solution was stirred at 40°C for 30 minutes. The excess epichlorohydrin was removed by distillation under reduced pressure. The residue was dissolved in 98% sulfuric acid (9.94 ml). It was necessary to control the temperature during the addition of concentrated sulfuric acid due to the high exothermic behaviour. Then, the solution was heated to 150 °C for 30 min. The resulting solution was cooled and added to ice (100 g) (high exothermic process), basified with NaOH 40% (high exothermic process) and the product was extracted with toluene. The toluene was evaporated and dried under vacuum to give 4 g (53 % yield) of product.

HPLC analysis of the product revealed a purity of intermediate (3) (A3VLX) of 89.79%, being accompanied with a high amount of impurities including:

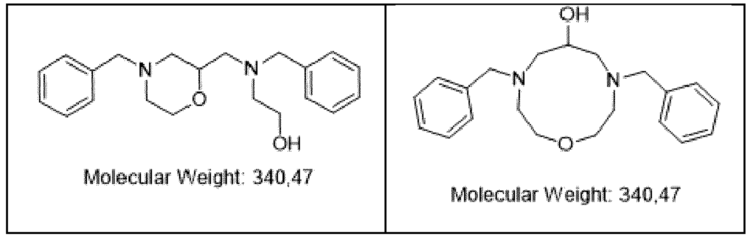

### Example 5. Formation of 4-benzyl-2-(chloromethyl)morpholine oxalate (oxalate salt of compound (3) or A3VLX oxalate)

The solvent from the organic phase mentioned in the Example 1 was subjected to partial distillation until a final volume of 500 ml, then 500 ml of acetone were added. The system was subjected again to partial distillation until a final volume of 500 ml, other 500 ml of acetone were added again and subjected to partial distillation until a final volume of 500 ml.

To this solution system, 59.4 g of oxalic acid (1 eq) solved in 200 ml of EtOH (2 vol) were carefully added. During the addition, precipitation of the oxalate salt was observed.

To the suspension formed, 500 ml of heptane (5 vol) were also added and the resulting slurry was filtered, washed with heptane and dried giving rise to 183 g of the oxalate salt of 4-Benzyl-2-(chloromethyl)morpholine (88% yield from (1)). HPLC purity 99.42%, DSC

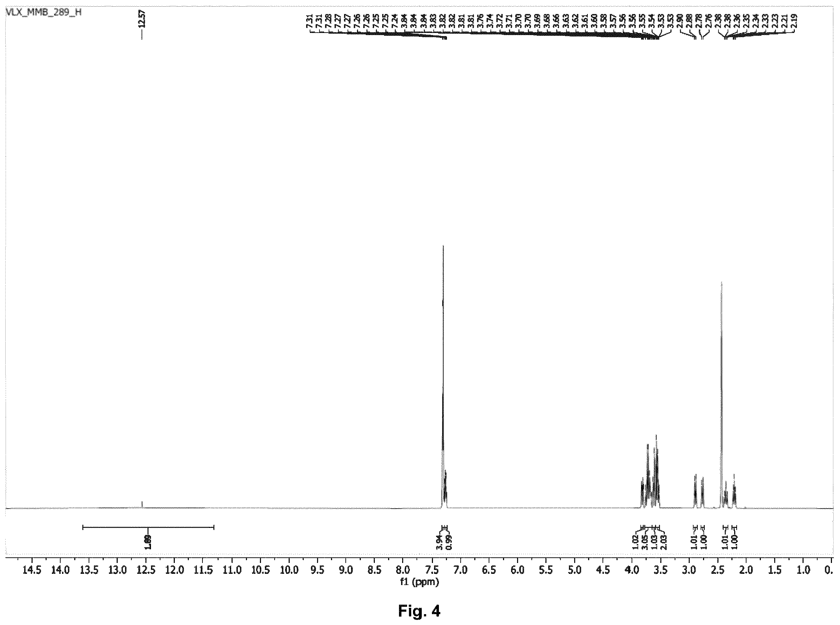

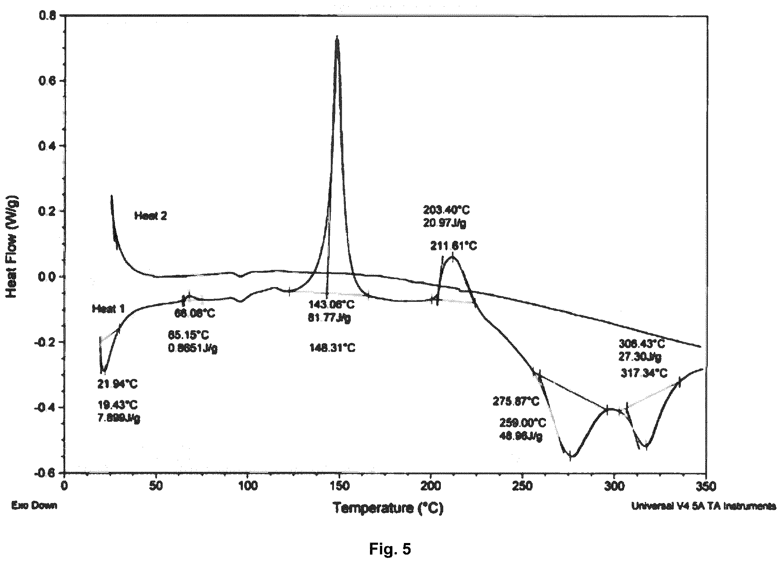

¹H NMR and DSC of 4-benzyl-2-(chloromethyl)morpholine oxalate are shown in Figures 4 and 5, respectively. ¹H NMR (500 MHz, DMSO-d6): δ 12,57 (bs, 2H); 7,31 (m, 4H); 7,24-7,28 (m, 1H); 3,81-3,84 (m, 1H); 3,66-3,74 (m, 3H); 3,60-3,63 (m, 1H); 3,53-3,58 (m, 2H); 2,88-2,90 (m, 1H); 2,76-2,78 (m, 1H); 2,36 (td, *J* = 11,7; 3,5 Hz, 1H); 2,21 (t, *J* = 10,8 Hz, 1H).

### Example 6. Formation of 4-benzyl-2-(chloromethyl)morpholine fumarate (fumarate salt of compound (3) or A3VLX fumarate)

A solution of 4-Benzyl-2-(chloromethyl)morpholine (3) (3 g) in isopropanol (9 ml, 3 Vol) was added to a solution of fumaric acid (1.85 g, 1.2 eq) in isopropanol (24 ml, 8 Vol) and the mixture was heated at 70°C.

The solution mixture was cooled down to room temperature, and afterwards, some solvent was vacuum distilled until some crystals appeared (normally, 3-4 vol). Once the precipitate was formed, heptane was added (5 vol) to facilitate stirring.

The suspension formed was filtered, rinsed with heptane and dried, giving rise to 3 g of a white solid of the fumarate salt of 4-Benzyl-2-(chloromethyl)morpholine (49% yield from (1)). HPLC purity 99.69%, DSC

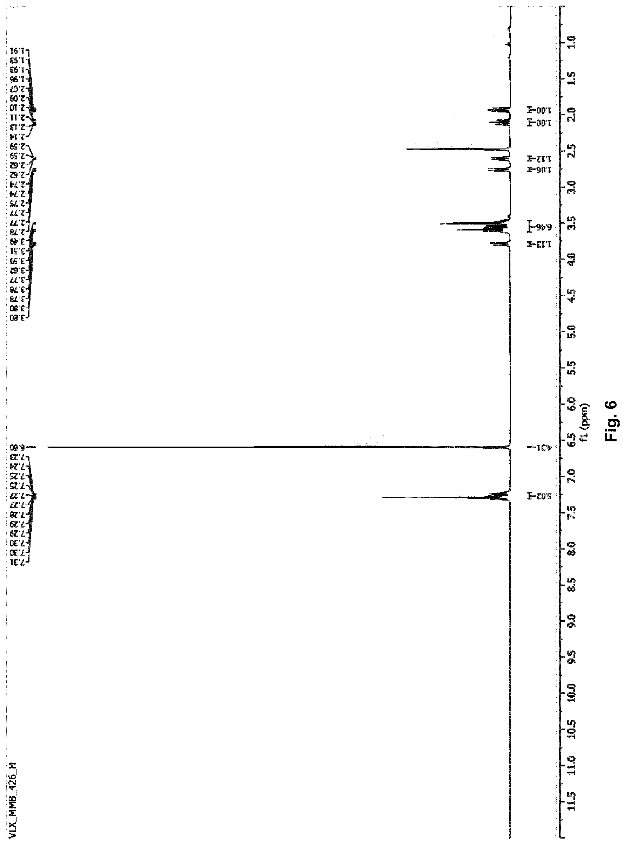

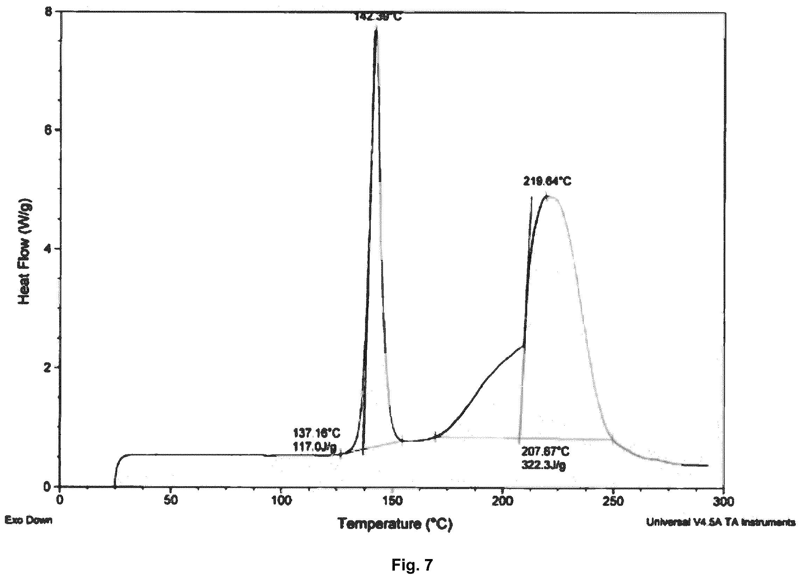

¹H NMR and DSC of 4-benzyl-2-(chloromethyl)morpholine fumarate are shown in Figures 6 and 7, respectively. ¹H NMR (400 MHz, DMSO-d6): δ 7,37-7,20 (m, 5H); 6,60 (s, 2H); 3,84-3,74 (m, 1H); 3,66-3,44 (m, 6H); 2,76 (dt, *J* = 11,3; 1,9 Hz, 1H); 2,60 (dd, *J* = 11,5; 2,1 Hz, 1H); 2,10 (td, *J* = 11,4; 3,3 Hz, 1H); 1,93 (dd, *J* = 11,2; 9,1 Hz, 1H).

### Example 7. Formation of 4-benzyl-2-(chloromethyl)morpholine tosylate (tosylate salt of compound (3) or A3VLX tosylate)

To a solution of 4-Benzyl-2-(chloromethyl)morpholine (3) (4 g) in acetone (40 ml, 10 Vol) at room temperature, a solution of p-toluenesulfonic acid (3.37 g, 1 eq) in EtOH (4 ml, 1 Vol) was added dropwise, and during the addition or after a while, a precipitate was formed.

The suspension formed was filtered, rinsed with acetone and dried, giving rise to 4.9 g of a white solid of the tosylate salt of 4-Benzyl-2-(chloromethyl)morpholine, (52% yield from (**1**)). HPLC purity 98.55%, DSC.

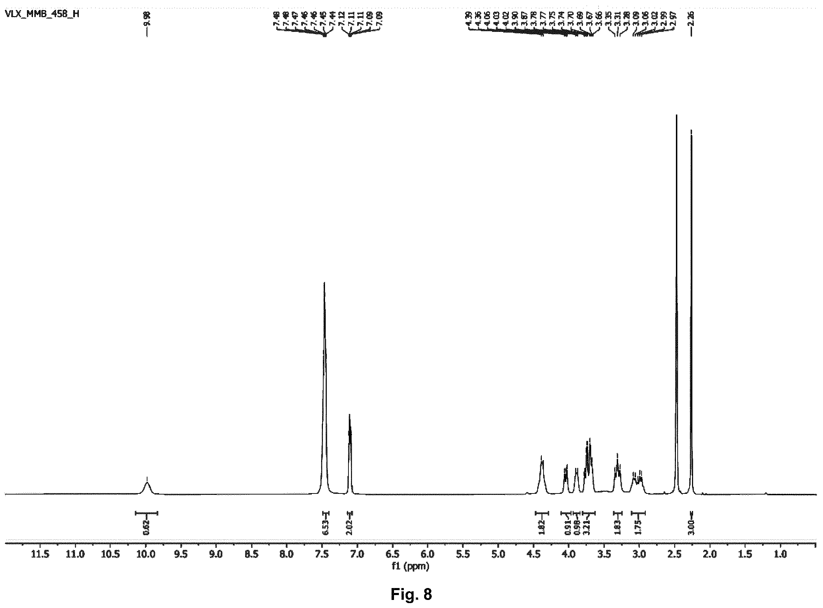

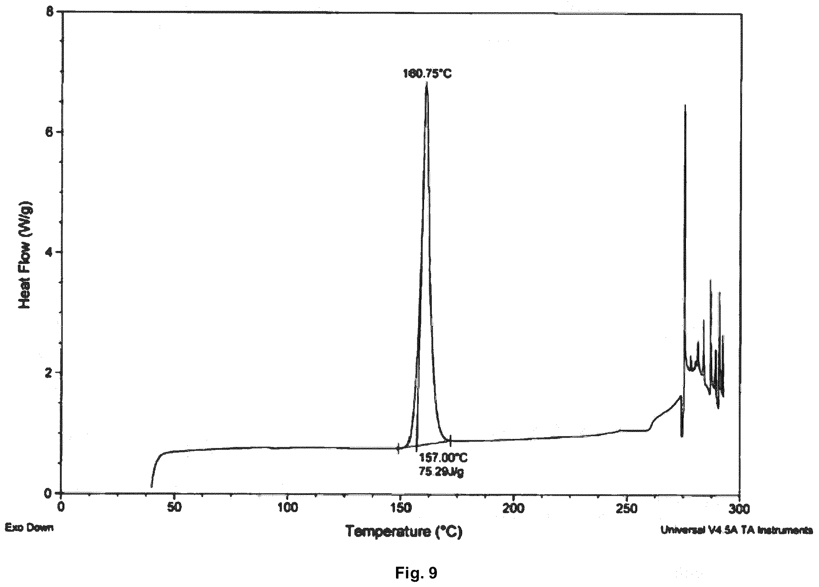

¹H NMR and DSC of 4-benzyl-2-(chloromethyl)morpholine tosylate are shown in Figures 8 and 9, respectively. ¹H NMR (400 MHz, DMSO-d6): δ 9 ,98 (s, 1H); 7,51-7,42 (m, 5H); 7,17-7,06 (m, 2H); 4,38 (m, 1H); 4,13-3,98 (m, 1H); 3,89 (m, 1H); 3,86-3,55 (m, 3H); 3,31 (m, 1H); 3,16-2,89 (m, 1H); 2,26 (s, 3H).

### Example 8. Formation of 4-Benzyl-2-((2-ethoxyphenoxy)methyl)morpholine (4) oxalate from 4-Benzyl-2-(chloromethyl)morpholine (3) oxalate.

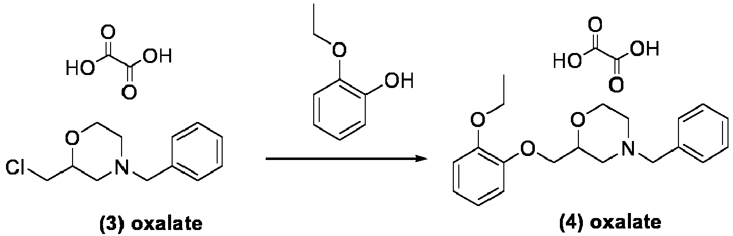

10 vol of dichloromethane, 5 vol of water and 15 vol of NaHCO₃ (7% w/w) were loaded onto the oxalate salt of intermediate (3). The mixture was stirred for 30 min and the phases were decanted. The organic phase was concentrated to residue.

11 vol of DMF, 4 eq of KOH and 4 eq of 2-ethoxyphenol were loaded onto the above residue and the reaction mixture was heated at 110°C for 2h.

Once the reaction was exhausted, the reaction mass was cooled down to room temperature and 20 vol of water and 10 vol of ethyl acetate were charged. The phases were decanted. The aqueous phase was extracted with 3 vol of ethyl acetate. The organic phases were combined and washed 3 times with 6 vol of water (to remove DMF). The organic phase was concentrated to 10 vol and 1 equivalent of oxalic acid dissolved in 1.5 vol of EtOH and 1 vol of ethyl acetate was dropped onto this solution. The suspension was filtered and washed with 2 vol of ethyl acetate. (62% yield from (1) and 83% from (3)).

### Example 9. Formation of Ethyl 2-((2-ethoxyphenoxy)methyl)morpholine-4-carboxylate (5) from 4-Benzyl-2-((2-ethoxyphenoxy)methyl)morpholine (4) oxalate.

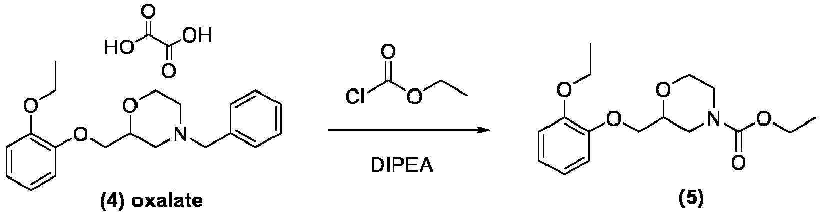

10 vol of dichloromethane, 5 vol of water and 15 vol of NaHCO₃ (7% w/w) were loaded onto the oxalate salt of intermediate (**4**). The mixture was stirred for 30 min and the phases were decanted. The organic phase was concentrated to residue.

6 vol of toluene, 1 eq of DIPEA and 2 eq of ethyl chloroformate were charged to the above residue and the reaction mixture was heated at 40°C for 2h.

Once the reaction was exhausted, the reaction mass was cooled down to room temperature and 10 vol of water were charged. The phases were decanted. The aqueous phase was extracted with 2 vol of toluene.

The organic phases were combined and concentrated to 2 vol. 12 vol of heptane were charged and a water/ice bath was set at 0/5°C. It began to crystallize. The suspension was filtered and washed with 2 vol of heptane. (66% yield from (**4**)).

### Example 10. Formation of 2-((2-ethoxyphenoxy)methyl)morpholine chloride from Ethyl 2-((2-ethoxyphenoxy)methyl)morpholine-4-carboxylate (5).

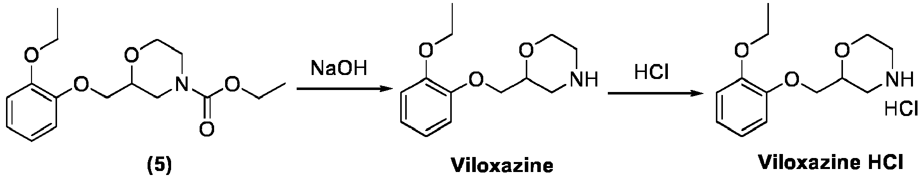

Intermediate (**5**) was dissolved in 9 vol of isopropanol, 3 eq of NaOH were charged and the mixture was heated at reflux for 16h.

Once the reaction was complete, the reaction mass was cooled to room temperature and 15 vol of ethyl acetate and 10 vol of water were charged. Phases were decanted. The aqueous phase was extracted with 4 vol of ethyl acetate. The organic phases were combined and concentrated to 4 vol. 10 vol of ethyl acetate were added and it was re-concentrated to 4 vol. 1.1 eq of HCI in IPA (5-6N) were dropped onto the product dissolved in ethyl acetate. The solution was cooled and the hydrochloride salt began to crystallize. The suspension was filtered and washed with 2 vol of ethyl acetate. (92% yield from (**5**)).

### Example 11. Formation of 4-Benzyl-2-((2-ethoxyphenoxy)methyl)morpholine (4) oxalate from 4-Benzyl-2-(chloromethyl)morpholine (3).

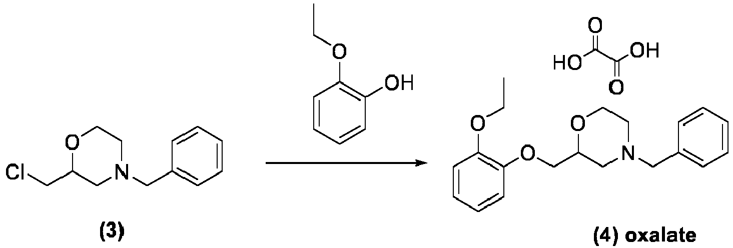

The organic phase in DCM that remains from the work-up of the intermediate (3) was exchanged for toluene: 2 drags of 6 vol of toluene were made, finally concentrating at 3 vol.

10 vol of DMSO, 1.1 eq of KOH and 1.1 eq of 2-ethoxyphenol were loaded onto that organic phase in toluene and the reaction mixture was heated at 100°C for 2h.

Once the reaction was exhausted, the reaction mass was cooled down to room temperature and 10 vol of water and 1 vol of toluene were charged. The phases were decanted. The aqueous phase was extracted with 2 vol of toluene. The organic phases were combined and concentrated to 4 vol (anhydration). 6 additional vols of toluene were loaded to make up to 10 vols. 2 vol of heptane were charged and 1 equivalent of oxalic acid dissolved in 3 vol of EtOH was dropped onto this solution of intermediate (4). The suspension was filtered and washed with 2 vol of toluene and 2 vol of heptane. (72% yield from (**1**)).

### Example 12. Formation of Ethyl 2-((2-ethoxyphenoxy)methyl)morpholine-4-carboxylate (5) from 4-Benzyl-2-((2-ethoxyphenoxy)methyl)morpholine (4).

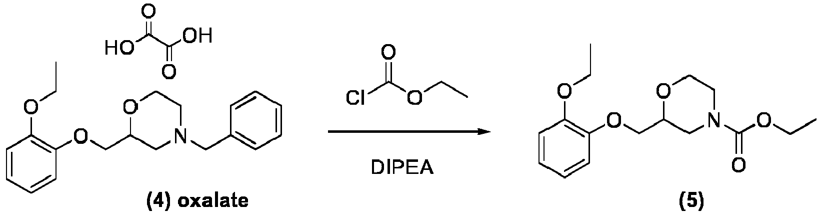

5 vol of dichloromethane, 8 vol of NaHCO₃ (5% w/w) were loaded onto the oxalate salt of intermediate (**4**). The mixture was stirred for 30 min and the phases were decanted. The organic phase was concentrated to 6 vol (anhydration).

1 eq of DIPEA and 1.5 eq of ethyl chloroformate were charged to intermediate (**4**) in 6 vol of DCM and the reaction mixture was heated at reflux for 2h.

Once the reaction was exhausted, 0.5 eq of DIPEA and 5 vol of water were added and the system was refluxed again for 1 h (hydrolysis of excess ethyl chloroformate).

The reaction mass was then cooled to room temperature and the phases were decanted. The aqueous phase was extracted with 2 vol of DCM. The organic phases were combined and concentrated to 2 vol. 18 vol of heptane were charged and it was concentrated to 18 vol. Another 3 vol of heptane were charged and it was concentrated again to 18 vol. It began to crystallize little by little. The suspension temperature was set to 0/5°C with an ice/water bath. The suspension was filtered and washed twice with 2 vol of cold heptane. (69% yield from (**4**)).

### Example 13. Formation of 2-((2-ethoxyphenoxy)methyl)morpholine chloride from Ethyl 2-((2-ethoxyphenoxy)methyl)morpholine-4-carboxylate (5).

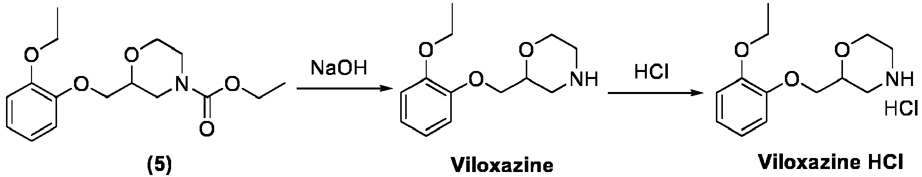

Intermediate (**5**) was dissolved in 9 vol of isopropanol, 3 eq of NaOH and 1 eq of water were charged and the mixture was heated under reflux for 16h.

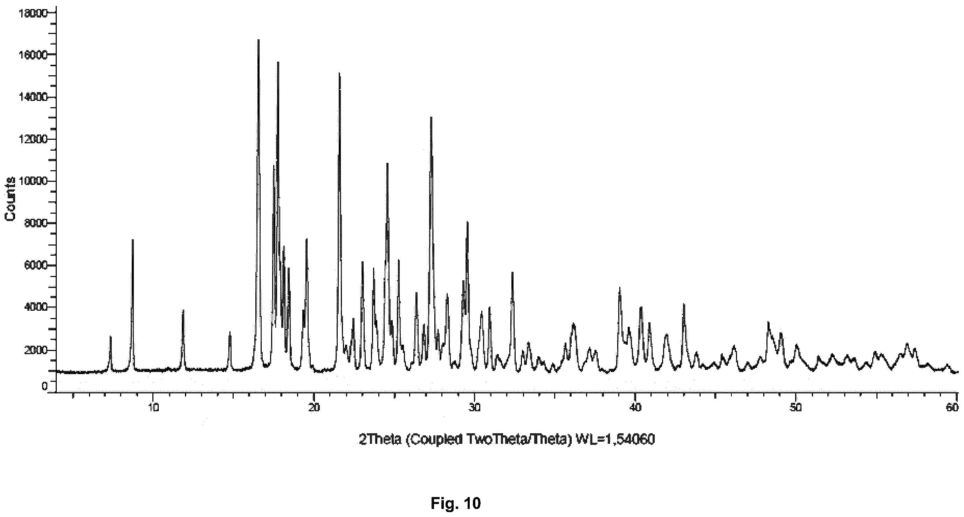

Once the reaction was complete, the reaction mass was cooled down to room temperature and 10 vol of water were charged. It was concentrated to 11 vol (to remove the IPA) and 10 vol of DCM were loaded. The phases were decanted. The organic phase was concentrated to 3 vol and stripped with 14 vol of ethyl acetate and concentrated to 10 vol. 1.1 eq of HCI 37% w/w were dropped onto the product dissolved in ethyl acetate and the product began to crystallize. The suspension was filtered and washed with 2 vol of ethyl acetate. (92% yield from (**5**)). HPLC purity (99.77%). See XRPD in Figure 10.

## Claims

1. A process for preparing a compound of general formula (III) or a salt or solvate thereof 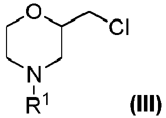 wherein R¹ is an amino protecting group,
said process comprising:
a) reacting epichlorhydrin with a compound of general formula (I) or a salt or solvate thereof 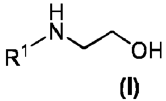 wherein R¹ is an amino protecting group,
in the presence of a Lewis acid and an organic solvent to obtain a reaction mixture comprising a compound of general formula (II) or a salt or solvate thereof 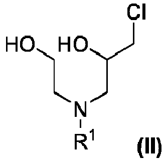 wherein R¹ is an amino protecting group,
b) mixing the reaction mixture with an aqueous solution of sulfuric acid to obtain a water-sulfuric phase comprising a sulfate salt of the compound of general formula (II); and
c) mixing the water-sulfuric phase comprising a sulfate salt of the compound of general formula (II) with concentrated sulfuric acid to obtain the compound of general formula (III) or a salt or solvate thereof.

2. The process according to claim 1, wherein the compound of general formula (III) is 4-benzyl-2-(chloromethyl)morpholine, which optionally is isolated in salt form, said salt being selected from the oxalate, fumarate or tosylate salt.

3. The process according to any one of claims 1 or 2 , wherein the Lewis acid is selected from the group consisting of LiClO₄, LiCI, LiBr, Lil·2H₂O, CsClO₄, Mg(ClO₄)₂, MgCl₂, MgBr₂, Mg(OAc)₂, CaCl₂, CaSO₄, TiCl₄, FeCI, FeCl₃, FeCl₃, CuCI, CuBr, CuCl₂, CuBr₂, ZnCl₂, ZnBr₂, Zn(OAc)₂, BBr₃, BF₃, BF₃·OEt₂, B(OPh)₃, B(OCH₃)₃, SnCI, TiCI, AlCl₃, SnCl₂, SnBr₂, SnCl₄, and SnBr₄.

4. The process according to claim 3, wherein the Lewis acid is selected from the group consisting of LiClO₄, LiCI, LiBr, Lil·2H₂O, CsClO₄, and Mg(ClO₄)₂.

5. The process according to claim 4, wherein the Lewis acid is LiClO₄.

6. The process according to any one of claims 1 to 5, wherein the compound of general formula (III) or a salt or solvate thereof is further converted into a compound of general formula (IV) or a salt or solvate thereof 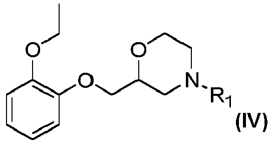 wherein R₁ is an amino protecting group,
by reaction with 2-ethoxyphenol in an organic solvent in presence of a base.

7. The process according to claim 6, wherein the compound of general formula (IV) is obtained in the form of an oxalate salt.

8. The process according to any one of claims 6 or 7, wherein the compound of general formula (IV) or a salt or solvate thereof is further converted into a compound of formula (V) or a salt or solvate thereof 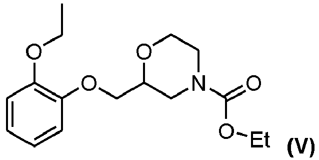 by reaction with ethyl chloroformate.

9. The process according to claim 8, wherein the compound of general formula (V) or a salt or solvate thereof is further converted into viloxazine or a salt or solvate thereof by a process comprising the addition of a base.

10. A process for the preparation of viloxazine comprising:
a) reacting epichlorohydrin with a compound of general formula (I) or a salt or solvate thereof 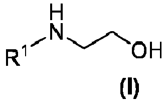 wherein R¹ is an amino protecting group,
in the presence of a Lewis acid and an organic solvent to obtain a reaction mixture comprising a compound of general formula (II) or a salt or solvate thereof 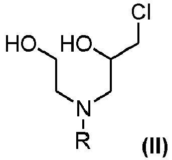 wherein R¹ is an amino protecting group,
b) mixing the reaction mixture with an aqueous solution of sulfuric acid to obtain a water-sulfuric phase comprising a sulfate salt of the compound of general formula (II); and
c) reacting the water-sulfuric phase comprising a sulfate salt of the compound of general formula (II) with concentrated sulfuric acid to obtain the compound of general formula (III) or a salt or solvate thereof;
d) reacting the compound of general formula (III) or a salt or solvate thereof with 2-ethoxyphenol in an organic solvent in presence of a base to obtain a compound of formula (IV) or a salt or solvate thereof 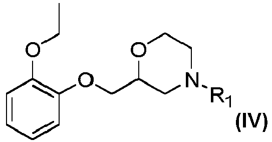 wherein R¹ an amino protecting group;
e) reacting the compound of general formula (IV) or a salt or solvate thereof with ethyl chloroformate to obtain a compound of formula (V) or a salt or solvate thereof 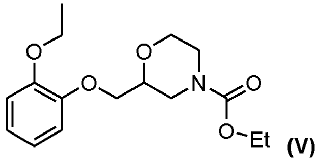 e) reacting the compound of formula (V) or a salt or solvate thereof with base to obtain viloxazine or a solvate thereof.

11. A process for the preparation of viloxazine according to claim 10, wherein the compound of general formula (III) is 4-benzyl-2-(chloromethyl)morpholine, which optionally is isolated in salt form, said salt being selected from the oxalate, fumarate or tosylate salt.

12. Salt of 4-benzyl-2-(chloromethyl)morpholine, said salt being selected from the oxalate, fumarate or tosylate salt.
